# EUROPEAN PATENT SPECIFICATION

(11) **EP 2 782 583 B1**
(45) Date of publication and mention of the grant of the patent: **25.09.2019**
(21) Application number: 12851204.3
(22) Date of filing: 21.11.2012
(51) Int. Cl.: A61K 31/56, A61K 31/26, A61K 31/275, A61P 25/32, A23L 33/10

(54) **METHODS AND COMPOSITIONS FOR REDUCING ALCOHOL TOXICITY**
VERFAHREN UND ZUSAMMENSETZUNGEN ZUR REDUZIERUNG EINER ALKOHOLTOXIZITÄT
PROCÉDÉS ET COMPOSITIONS POUR RÉDUIRE LA TOXICITÉ DE L'ALCOOL

(30) Priority: 22.11.2011 US 201161562559 P; 09.12.2011 US 201161568721 P
(43) Date of publication of application: 01.10.2014
(73) Proprietor: The John Hopkins University, Baltimore, MD 21218 (US)
(72) Inventor: TALALAY, Paul, Baltimore, MD (US); USHIDA, Yusuke, Baltimore, MD (US)
(74) Representative: Valea AB
(86) International application number: PCT/US2012/066340
(87) International publication number: WO 2013/078371

(56) References cited:
- WO-A1-2007/016953
- US-A1- 2005 271 754
- US-A1- 2008 311 276
- US-A1- 2011 065 659
- DATABASE WPI Week 200267 Thomson Scientific, London, GB; AN 2002-624431 XP002750844, & KR 2002 0014978 A (KIM G S) 27 February 2002 (2002-02-27)
- EUN JU CHOI ET AL: "Four flavonoids from Echinosophora koreensis and their effects on alcohol metabolizing enzymes", ARCHIVES OF PHARMACAL RESEARCH, vol. 32, no. 6, 1 June 2009 (2009-06-01), pages 851-855, XP055048950, ISSN: 0253-6269, DOI: 10.1007/s12272-009-1606-2
- THIMMULAPPA ET AL.: 'Identification of Nrf2-regulated Genes Induced by the Chemopreventive Agent Sulforaphane by Oligonucleotide Microarray' CANCER RESEARCH vol. 62, 15 September 2002, pages 5196 - 5203, XP002471401
- HEAVEY ET AL.: 'Colorectal Cancer and the Relationship Between Genes and the Environment' NUTRITION AND CANCER vol. 48, no. 2, 2004, pages 124 - 141, XP008174354
- SERGEY M. ZIMATKIN ET AL: "Enzymatic Mechanisms of Ethanol Oxidation in the Brain", ALCOHOLISM: CLINICAL AND EXPERIMENTAL RESEARCH., vol. 30, no. 9, 1 September 2006 (2006-09-01), pages 1500-1505, XP055369802, US ISSN: 0145-6008, DOI: 10.1111/j.1530-0277.2006.00181.x
- The Jousrr ET AL: "Phenethyl Isothiocyanate, a New Dietary Liver Aldehyde Dehydrogenase Inhibitor1", , 29 June 1995 (1995-06-29), XP055369803, Retrieved from the Internet: URL:http://jpet.aspetjournals.org/content/ 275/1/79.full-text.pdf

## Description

This invention was made with government support under grant nos. awarded by . The government has certain rights in the invention.

### CROSS-REFERENCE TO RELATED APPLICATIONS

This application claims the priority of U.S. Provisional Patent Application No. 61/568,721, filed December 9, 2011.

### TECHNICAL AREA

The present invention comprises methods and compositions for reducing alcohol toxicity, particularly by modulating the amount of acetaldehyde present in a subject.

### BACKGROUND

Modest consumption of ethyl alcohol is believed to have beneficial effects on human health including improving cardiovascular function, but large amounts of ethanol can damage the liver, heart and pancreas, and affect the nervous system (Crabb 2004). Ethanol is metabolized to acetaldehyde, which is mutagenic and carcinogenic, and is responsible for the unpleasant symptoms of heavy alcohol consumption and health damage. Acetaldehyde is metabolized to non-toxic acetate by acetaldehyde dehydrogenases, predominantly the cytosolic ALDH1 and mitochondrial ALDH2 (Impraim 1982). Polymorphism of ALDH2 is widespread, and the occurrence rate is particularly high among East Asians such as Chinese, Japanese and Koreans. Approximately 40% of these populations have a mutation in ALDH2 (the mutated version of which is henceforth denoted as ALDH2*2), which has much lower or no activity, and such persons are highly sensitive to the consumption of alcohol (Yoshida 1984; Eng MY 2007). These individuals display characteristic responses to drinking alcohol including facial flushing, nausea, and tachycardia. Moreover, these individuals are much more vulnerable to damage from acetaldehyde including much higher risks of developing cancer and other serious diseases (Harada 1981). In particular, the risk of developing esophageal squamous cell (ESC) cancer, which is a common disease in middle aged Japanese men, is often associated with the polymorphism of ALDH2 (Brooks 2009; Oze 2011). The risk of ESC cancer is dramatically increased in individuals with ALDH2*2 who drink alcohol compared to those with non-mutated or wild-type ALDH2 (Yokoyama 2003). Alcohol and the associated accumulation of acetaldehyde that results from alcohol consumption are considered to be factors commonly responsible for the development of esophageal cancer. US 2011/065659 discloses the use of ginsenoside compositions for reducing acetaldehyde concentrations, for preventing or ameliorating a symptom of elevated acetaldehyde concentration, or for reducing the risk of disorders caused by the intake of alcohol. KR 2002 0014978 discloses the effect of aromatics, such as flavonoids, in promoting alcohol metabolism. Eun Ju Choi et al. (Arch Pharm Res, vol. 32, no. 6, June 1 2009, p. 851-855) discloses the effects of some flavonoids on the activities of ALD and ALDH and suggest their use in treating hangover. WO 2007/016953 discloses a food composition or dietary supplementation for slowing down the process of ethanol metabolism to prevent the accumulation or acetaldehyde.

What is needed are methods and compositions for increasing the amount or expression of acetaldehyde dehydrogenase enzymes or acetaldehyde dehydrogenase enzymatic activity in a subject, thereby reducing the levels of acetaldehyde accumulation in the subject and alleviating, preventing, or reducing the toxicity associated with acetaldehyde accumulation. This is particularly needed in populations with mutated ALDH or in those that consume alcohol or both.

### SUMMARY

In accordance with the purpose(s) of the invention, as embodied and broadly described herein, the invention, in one aspect, relates to methods and compositions for increasing the amount of acetaldehyde dehydrogenase enzymes (ALDH) and/or increasing ALDH catalytic enzyme activity present in a subject. Disclosed herein are methods and compositions for reducing peak blood levels of acetaldehyde and/or increasing the rate of metabolism of acetaldehyde in persons who consume or have consumed alcohol

Disclosed herein are methods for reducing the level of acetaldehyde in a subject comprising administering an effective amount of a composition comprising a compound that modulates the expression of acetaldehyde dehydrogenase (ALDH) in at least one cell of a subject.

Disclosed herein are methods for reducing the level of acetaldehyde in a subject comprising administering an effective amount of a composition comprising a compound that increases the amount of acetaldehyde dehydrogenase activity in at least one cell of a subject.

Disclosed herein are methods for reducing or preventing ethanol toxicity due to acetaldehyde accumulation in a subject who consumes ethanol, comprising administering an effective amount of a compound to increase the expression of acetaldehyde dehydrogenase in at least one cell of a subject.

Disclosed herein are methods for reducing or preventing ethanol toxicity due to acetaldehyde accumulation in a subject who consumes ethanol, comprising administering an effective amount of a compound to increase the amount of acetaldehyde dehydrogenase activity or the amount of ALDH enzymes in at least one cell of a subject.

Disclosed herein are methods for increasing the gene expression of at least one member of the acetaldehyde dehydrogenase superfamily in a subject comprising administering an effective amount of a phase 2 inducer, thereby increasing the gene expression of at least one member of the acetaldehyde dehydrogenase superfamily.

Disclosed herein are methods for increasing the amount of at least one member of the acetaldehyde dehydrogenase superfamily or the enzymatic activity of at least one member of the acetaldehyde dehydrogenase superfamily in a subject, comprising administering an effective amount of a phase 2 inducer, thereby increasing the amount of at least one member of the acetaldehyde dehydrogenase superfamily or the enzymatic activity of at least one member of the acetaldehyde dehydrogenase superfamily.

Disclosed herein are methods for increasing the amount of at least one member of the acetaldehyde dehydrogenase superfamily or the enzymatic activity of at least one member of the acetaldehyde dehydrogenase superfamily in a subject comprising administering an effective amount of a compound that induces NQO1, thereby increasing the amount of at least one member of the acetaldehyde dehydrogenase superfamily or the enzymatic activity of at least one member of the acetaldehyde dehydrogenase superfamily.

Disclosed herein are methods for increasing the amount of at least one member of the acetaldehyde dehydrogenase superfamily or the enzymatic activity of at least one member of the acetaldehyde dehydrogenase superfamily in a subject comprising administering an effective amount of a compound that upregulates at least one step in the Keap1/Nrf2/ARE transcription pathway, thereby increasing the amount of at least one member of the acetaldehyde dehydrogenase superfamily or the enzymatic activity of at least one member of the acetaldehyde dehydrogenase superfamily.

Compositions of the of the present invention may comprise a pharmaceutical composition, a nutraceutical composition, a natural product composition, a medical food, a medicament, a nutritional supplement, or a composition comprising excipients, diluents, enzymes, cofactors, and delivery vehicle additives.

Disclosed herein are compositions comprising one or more precursor compounds, such as glucosinolate, for the treatment of alcohol toxicity or acute or long-term toxicity associated with acetaldehyde.

Disclosed herein are compositions comprising one or more enzymes for which the compound provided in the composition is a substrate molecule of the one or more enzymes.

Disclosed herein are compositions comprising a phase 2 inducer. A composition of the present invention may include, but is not limited to, one or more of sulforaphane, erucin, iberin, benzyl ITC, phenethyl ITC, propyl ITC, hexyl ITC, 4RBITC, TP-225, celastrol, withaferin A, TBE-31, HBB-2, HBB-4, BNF, pinostrobin, tectochrisin, kaempferide, resveratrol and zerumbone, or a combination thereof.

Disclosed herein are compositions comprising a compound that is present as a structure represented by a formula shown below, or a subgroup or pharmaceutically acceptable salt thereof. 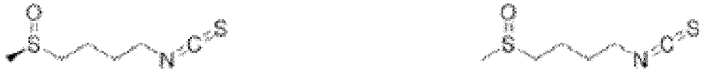 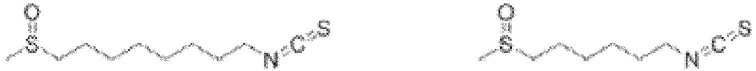 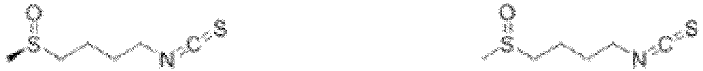 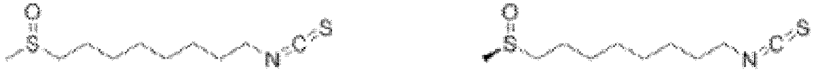 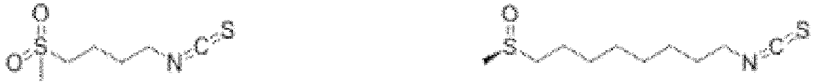 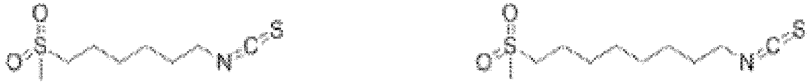 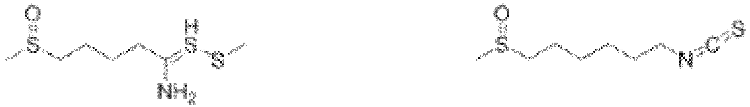 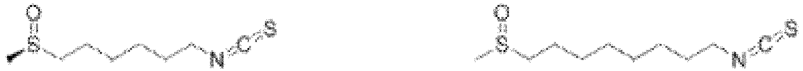 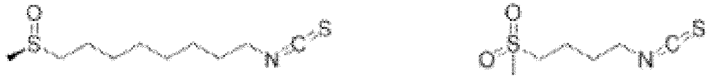 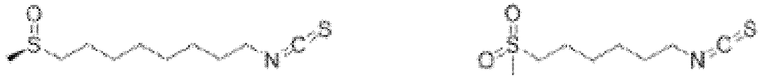 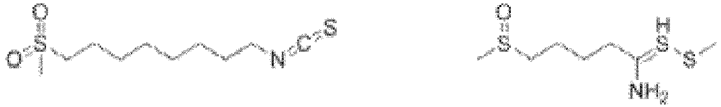 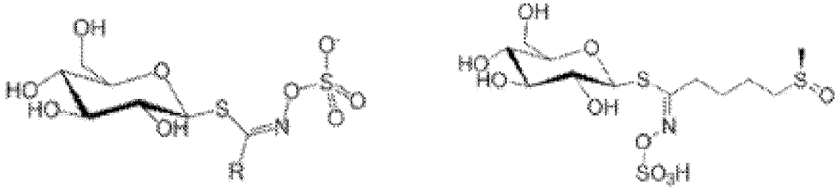 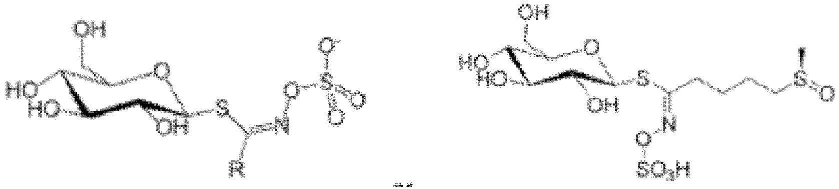 or

A composition disclosed herein may comprise a pharmaceutical composition, a nutraceutical composition, a natural product composition, a medical food, a medicament, a nutritional supplement, or a composition comprising excipients, diluents, enzymes, cofactors, and delivery vehicle additives.

Disclosed herein is a method for determining effectiveness of a compound in treating ethanol toxicity due to acetaldehyde accumulation, comprising, optionally treating cells in the assay with ethanol, administering to a first cell an effective amount of a compound to be tested, comparing the response of the first cell with the response of an identical cell that was treated with a compound known to increase the amount and/or enzymatic activity of at least one acetaldehyde dehydrogenase, and determining whether or not the tested compound reduces ethanol toxicity in cells.

Disclosed herein are medical foods used to treat acetaldehyde accumulation, which may result from ethanol consumption.

Disclosed herein are compositions comprising dietary or nutritional supplements used to treat acetaldehyde accumulation, which may result from ethanol consumption.

Disclosed herein are compounds derived from natural or synthetic sources which may be used in a composition to treat acetaldehyde accumulation, which may result from ethanol consumption.

### DESCRIPTION OF FIGURES

The accompanying Figures, which are incorporated in and constitute a part of this specification, illustrate several aspects and together with the description serve to explain the principles of the invention.

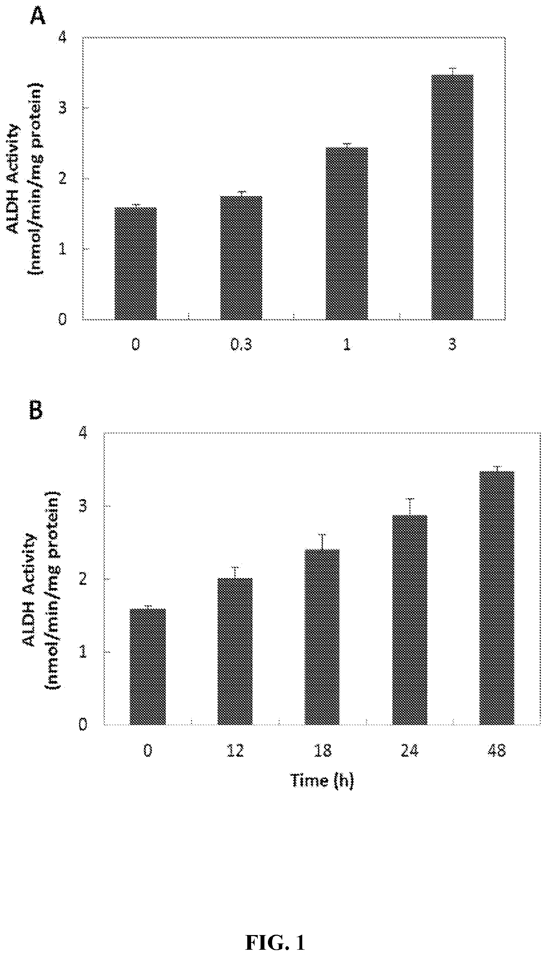

Figure 1 shows the effect of sulforaphane on ALDH activity in Hepa1c1c7 cells. Hepa1c1c7 cells were treated with a series concentration of sulforaphane (0.3, 1.0 or 0.3 µM) for 48 h (A) or with 3.0 µM sulforaphane for 0, 12, 18, 24 or 48 h (B). ALDH activity was measured in supernatant fraction of cell homogenates using propionaldehyde as a substrate. Means ± S.E.M. are shown (n = 8).

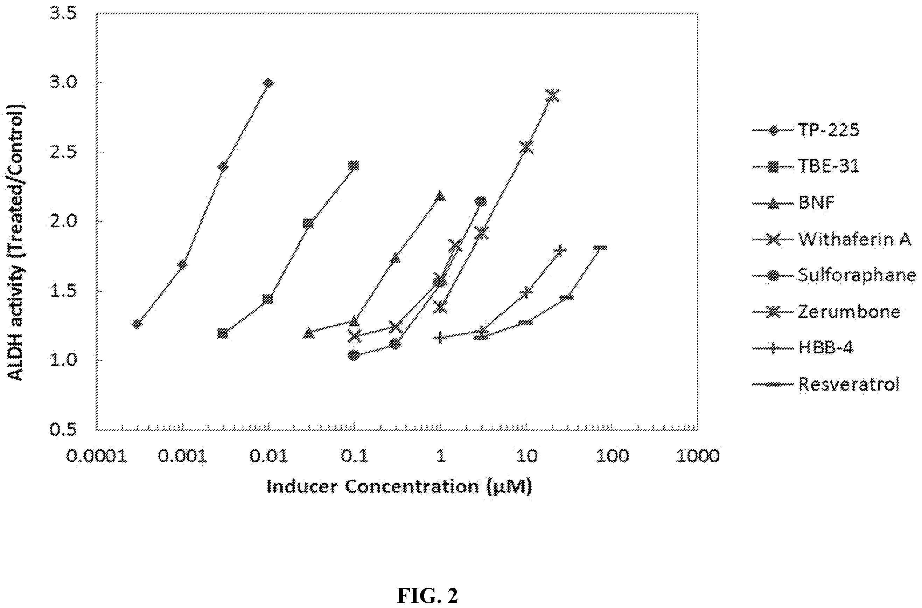

Figure 2 shows the effect of phase 2 inducers with distinct chemical structures on ALDH activity in Hepa1c1c7 cells. Hepa1c1c7 cells were treated with a stepwise concentration of phase 2 inducers for 48 h. ALDH activity was measured in supernatant fraction of cell homogenate by using propionaldehyde as a substrate and then calculated as a relative value (Treated/Control). Results are shown as average values of 8 replicate plates. The standard deviations in each case were between 5 and 10% of the observed values.

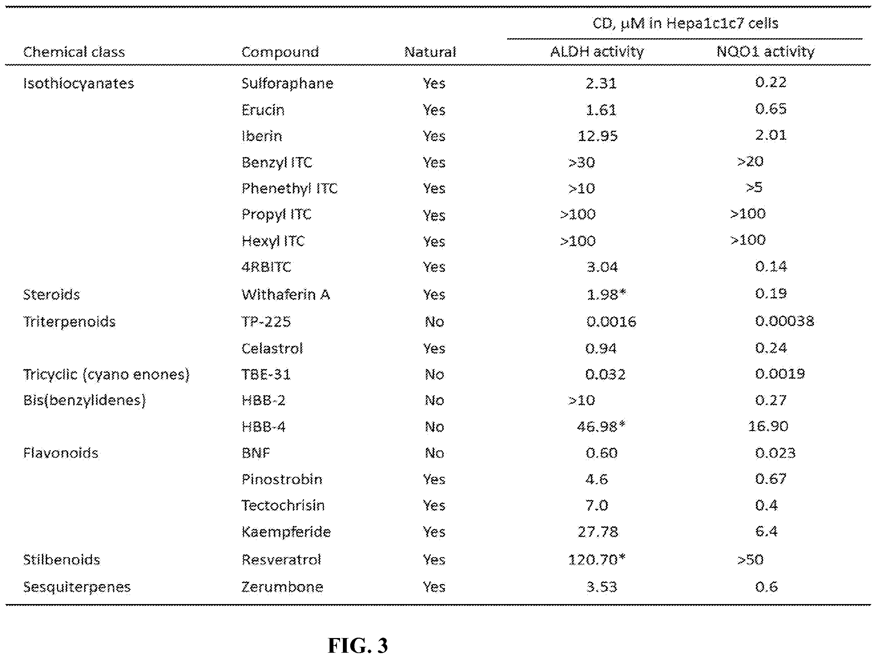

Figure 3 shows potencies of compounds for induction of ALDH and NQO1 in Hepa1c1c7 cells (CD: the concentration required to double the enzymatic activity, n=8, *: Extrapolated value).

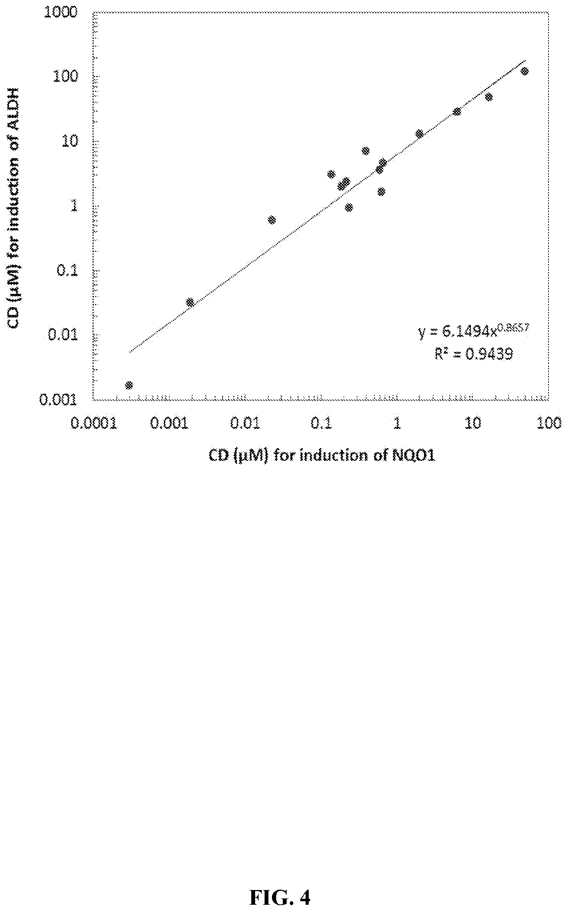

Figure 4 shows the relationship of potencies of 15 inducers for induction of ALDH and NQO1. The concentration required to double the enzymatic activity (CD) was calculated as potency of each inducers for induction of ALDH and NQO1. The CD values of 15 inducers are plotted on the double logarithmic graph.

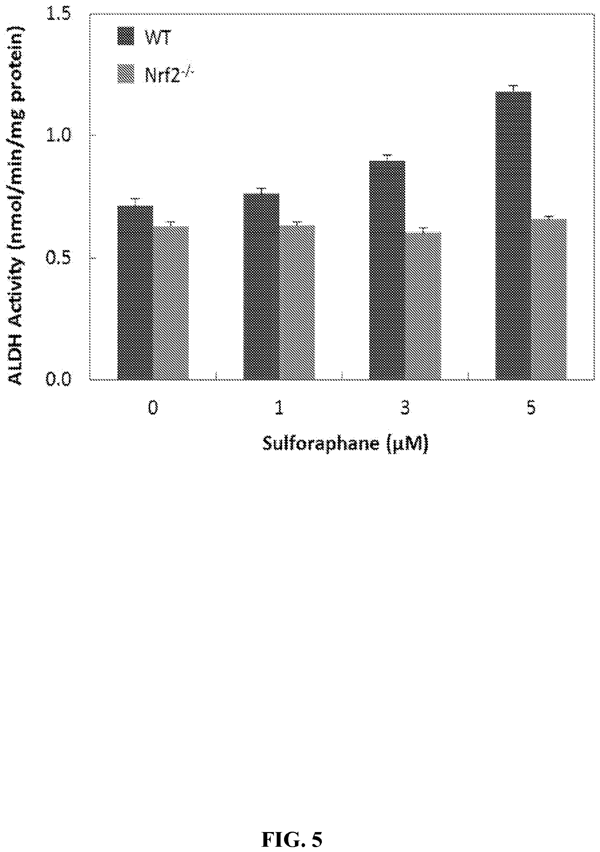

Figure 5 shows the effect of sulforaphane on ALDH activity in WT and Nrf2^{-/-} MEFs. Mouse embryonic fibroblasts isolated from wild-type (WT) or Nrf2 knock-out (Nrf2^{-/-}) mice were treated with a series concentration of sulforaphane for 48 h. ALDH activities were measured in supernatant fraction of cell homogenates by using propionaldehyde as a substrate. Means ± S.E.M. are shown (n = 8).

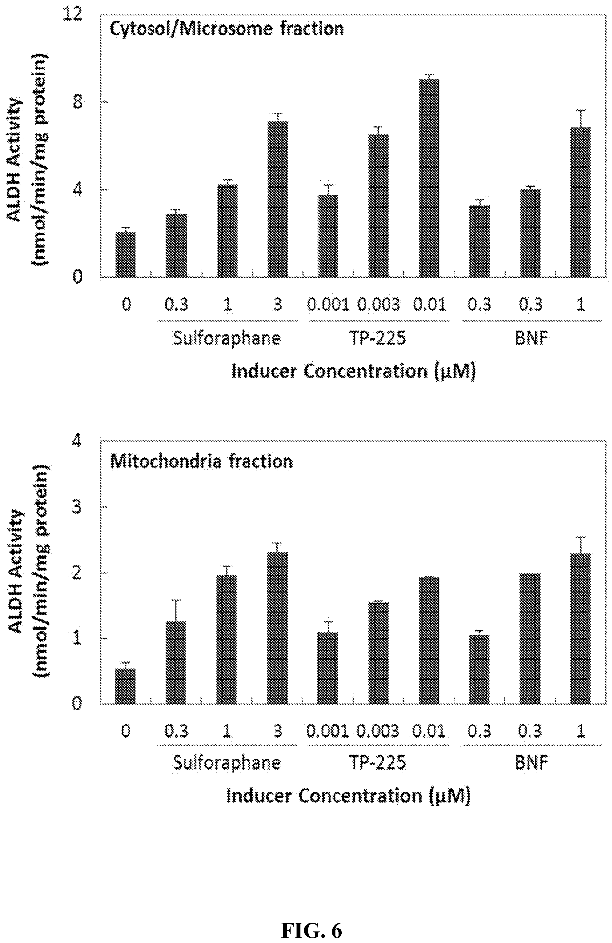

Figure 6 shows the effect of sulforaphane on ALDH activities in subcellular fractions Hepa1c1c7 cells. Hepa1c1c7 cells were treated with representative phase 2 inducers that belong to distinct chemical classes. After 48 h of treatment, cells were scraped, homogenized and fractionated into cytosolic/microsomal- and mitochondrial-fractions. ALDH activities in both fractions were determined by using propinoaldehyde as a substrate. Means ± S.E.M. are shown (n = 4).

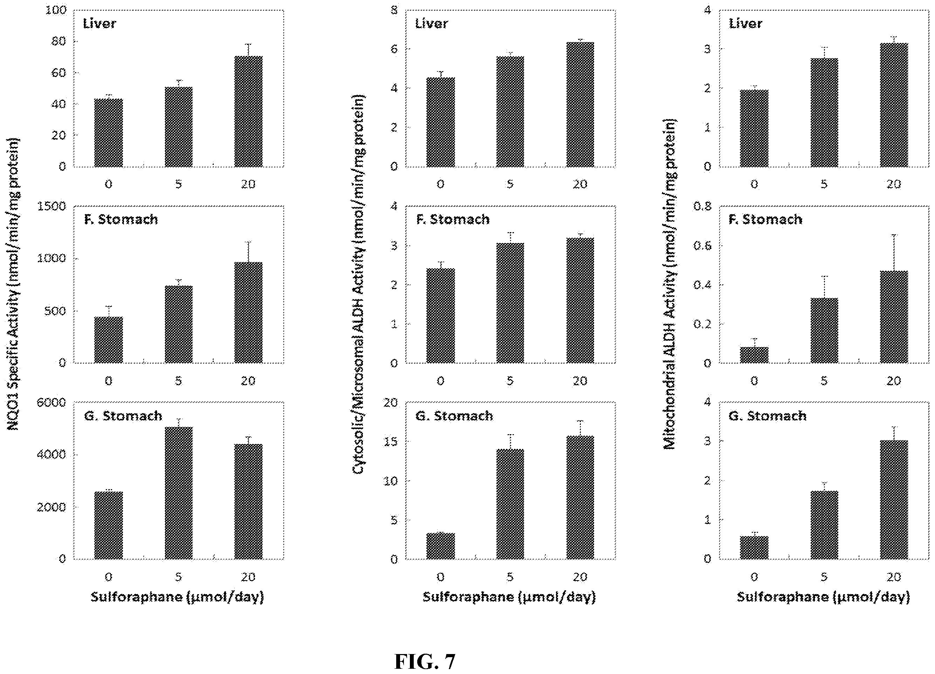

Figure 7 shows the effects of sulforaphane on enzyme activities of NQO1 and ALDH in mouse organs.

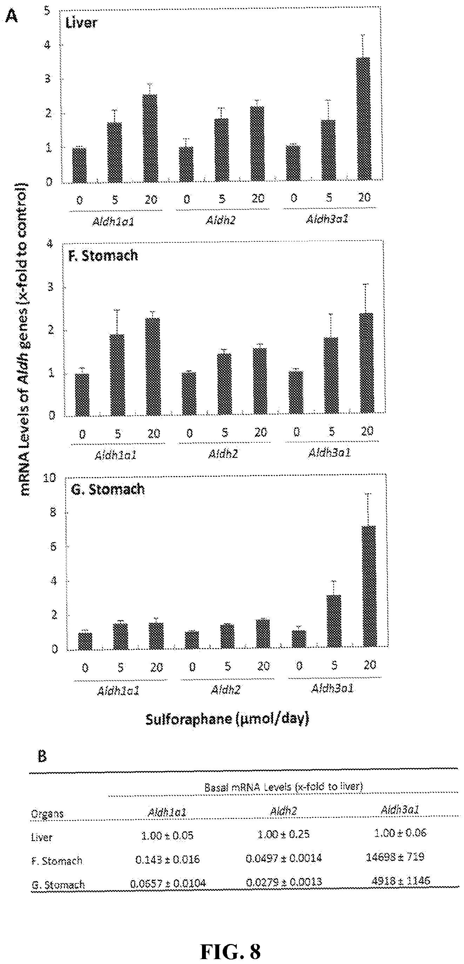

Figure 8 shows the effects of sulforaphane on mRNA levels of ALDH genes in mouse organs. The comparative 2^{-ΔΔCT} method was used to compare mRNA levels in organs collected from CD-1 mice that received sulforaphane containing diets (0, 5 or 20 µmol/day) for 1 week, β-actin was used as an endogenous control for all target genes, and the values are represented as the relative fold change in the mRNA levels of the SF-fed versus control animals. Intake of sulforaphane increased mRNA levels *Aldh* genes in mouse organs (A). The basal mRNA levels of *Aldhs* were significantly varied among organs (B). Error bars represent the standard deviation (SD) of the mean of the average 2^{-ΔΔCT} values for each group.

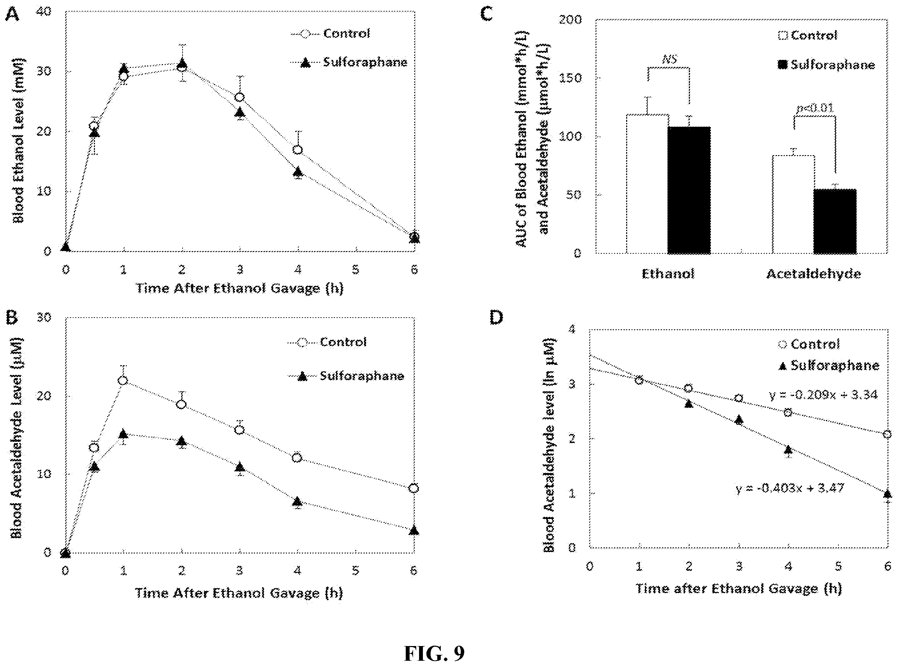

Figure 9 shows the effect of sulforaphane on ethanol and acetaldehyde levels in mouse blood after ethanol gavage. After feeding control diet or sulforaphane containing diet (20 µmol/3 g/day) for 1 week (n = 9 in each), CD-1 mice were orally administered ethanol at a dose of 2.0 g/kg. Intake of sulforaphane remarkably lowered acetaldehyde levels but not ethanol levels in blood (A and B). Calculated AUC values also indicated that sulforaphane affected principally affected acetaldehyde levels (C). Pharmacokinetics analysis revealed that elimination of blood acetaldehyde was accelerated in SF fed mice (D). Means ± S.E.M. are shown.

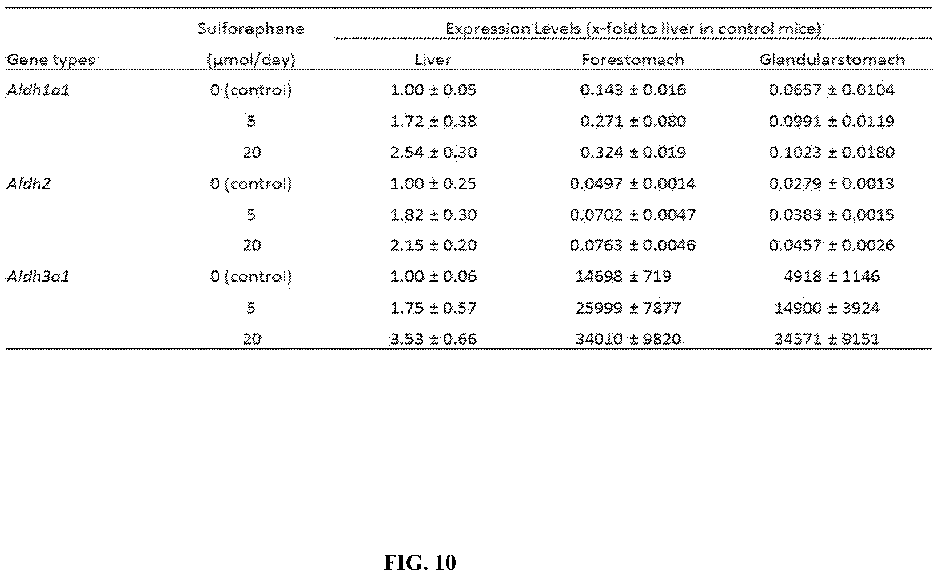

Figure 10 shows the effects of sulforaphane on mRNA levels of ALDH genes in mouse organs. The comparative 2^{-ΔΔCT} method was used to compare mRNA levels in organs collected from CD-1 mice that received sulforaphane containing diets (0, 5 or 20 µmol/day) for 1 week. β-actin was used as an endogenous control for all target genes, and the values are represented as the relative fold change in the mRNA levels to liver in control mice. Intake of sulforaphane increased mRNA levels *Aldh* genes in mouse organs. The basal mRNA levels of ALDHs were significantly varied among organs. Data represent the mean ± standard deviation (SD).

### DETAILED DESCRIPTION

The present invention can be understood more readily by reference to the following detailed description of the invention, the figures and the examples included herein.

The present invention comprises methods and compositions for modulating the effects of acetaldehyde in a subject or in at least one cell of a subject. The presence of toxic aldehydes, such as acetaldehyde, in a subject, such as a human, causes acute cellular damage and long-term exposure to acetaldehyde causes chronic responses and may lead to cellular changes such as cancer, such as esophageal cancer. Compositions and methods of the present invention are useful for modulating the effects of aldehyde toxicity in subjects.

### METHODS

Disclosed herein methods for reducing the level of acetaldehyde in a cell, in at least one cell of a subject or in a subject by administering an effective amount of at least one compound disclosed herein, wherein after administration, the level of acetaldehyde in at least one cell, in the subject or in at least one cell of a subject, is reduced. Reduction of acetaldehyde level in a subject may lead to reduced toxicity due to the effects of acetaldehyde in the subject, reduced toxicity due to ethanol consumption, and may modulate the acute and long-term effects of alcohol consumption. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound may comprise an isothiocyanate (ITC), a glucosinolate, sulforaphane, a sulforaphane derivative, a sulforaphane analog, erucin, iberin, benzyl-ITC, phenethyl-ITC, propyl-ITC, hexyl-ITC, 4RB-ITC (4-rhamno benzyl-ITC), withaferin A, a steroid, triterpenoids, TP-225, celastrol, tricyclic (cyano enones), TBE-31, bis(benzylidenes), HBB-2, HBB-4, a flavonoid, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

The present document comprises a method for reducing peak blood levels of acetaldehyde and increasing the rate of catabolism of acetaldehyde in a subject, comprising, administering an effective amount of a compound that modulates the expression, amount, or enzymatic activity of an acetaldehyde dehydrogenase found in at least one cell of a subject or a subject, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, sulforaphane, a sulforaphane derivative, a sulforaphane analog, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

A method for reducing acetaldehyde levels and/or increasing the rate of catabolism of acetaldehyde, comprising, contacting at least one cell with an effective amount of a compound that modulates the expression, amount, or enzymatic activity of an acetaldehyde dehydrogenase found in at least one cell. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, sulforaphane, a sulforaphane derivative, a sulforaphane analog, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2). In an aspect, acetaldehyde dehydrogenase is located in the cytosolic fraction of a cell, microsomal fraction of a cell, mitochondria or in some or all locations. In an aspect, expression of acetaldehyde dehydrogenase increases in the liver, the forestomach, the glandular stomach, the small intestine or a combination thereof. In an aspect, modulating the expression of acetaldehyde dehydrogenase and/or modulating the activity of acetaldehyde dehydrogenase reduces, lowers, and/or prevents alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for treating a subject diagnosed with an undesired level of acetaldehyde, comprising administering an effective amount of a composition comprising a compound disclosed herein that modulates the expression of acetaldehyde dehydrogenase and/or that modulates the activity of acetaldehyde dehydrogenase, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, sulforaphane, a sulforaphane derivative, a sulforaphane analog, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for treating a subject having an undesired level of acetaldehyde, comprising administering an effective amount of a composition comprising a compound disclosed herein that modulates the expression of acetaldehyde dehydrogenase and/or that modulates the activity of acetaldehyde dehydrogenase, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, sulforaphane, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for reducing the level of acetaldehyde in a subject comprising administering an effective amount of a composition comprising a compound disclosed herein that modulates the expression of acetaldehyde dehydrogenase in at least one cell of a subject, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, sulforaphane, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for reducing the level of acetaldehyde in a subject comprising administering an effective amount of a composition comprising a compound disclosed herein that increases the amount of acetaldehyde dehydrogenase activity in at least one cell of a subject, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutation of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for reducing or preventing ethanol toxicity due to acetaldehyde accumulation in a subject who consumes ethanol, comprising administering a compound disclosed herein to increase the expression of acetaldehyde dehydrogenase in at least one cell of a subject, wherein after administration, the level of acetaldehyde decreases in at least one cell or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing in many tissues levels in the subject, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1 B1 or a combination thereof. In an aspect, ALDH2 is mutated (ALDH2*2). In an aspect, acetaldehyde dehydrogenase is located in the cytosolic fraction of a cell, microsomal fraction of a cell, mitochondria or in some or all locations. In an aspect, expression of acetaldehyde dehydrogenase increases in the liver, the forestomach, the glandular stomach, the small intestine or a combination thereof. In an aspect, modulating the expression of acetaldehyde dehydrogenase and/or modulating the activity of acetaldehyde dehydrogenase reduces, lowers, and/or prevents alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for reducing or preventing ethanol toxicity due to acetaldehyde accumulation in a subject who consumes ethanol, comprising administering an effective amount of a compound disclosed herein to increase the amount of acetaldehyde dehydrogenase activity in at least one cell of a subject, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldhla1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutations of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for increasing the gene expression of at least one member of the acetaldehyde dehydrogenase superfamily, comprising administering to at least one cell, to at least one cell of a subject, or to a subject, an effective amount of a phase 2 inducer, including, but not limited to the compounds disclosed herein, to increase the gene expression of at least one member of the acetaldehyde dehydrogenase superfamily, as measured by assays known to those of skill in the art, in at least one cell, in a subject or in at least one cell of a subject, wherein after administration, the level of acetaldehyde decreases in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutations of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

A method of the present document comprises a method of increasing the amount of at least one member of the acetaldehyde dehydrogenase superfamily or increasing the enzymatic activity of at least one member of the acetaldehyde dehydrogenase superfamily, comprising administering to at least one cell, to at least one cell of a subject, or to a subject, an effective amount of a compound, including, but not limited to the compounds disclosed herein, that induces NQO1 to increase the amount of at least one member of the acetaldehyde dehydrogenase superfamily or increase the enzymatic activity of at least one member of the acetaldehyde dehydrogenase superfamily, wherein after administration, the level of acetaldehyde decreases in at least one cell, in at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutations of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

A method of the present document comprises a method for increasing the amount of at least one member of the acetaldehyde dehydrogenase superfamily, comprising, administering to at least one cell, to at least one cell of a subject, or to a subject, an effective amount of a compound, including, but not limited to the compounds disclosed herein, that upregulates at least one step in the Keap1/Nrf2/ARE transcription pathway to increase the amount of at least one member of the acetaldehyde dehydrogenase superfamily, wherein after administration, the level of acetaldehyde decreases in at least one cell, at least one cell of the subject or in the subject. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutations of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed methods are directed to modulating acetaldehyde dehydrogenase such that expression, amount, or activity of these enzymes are increased, thus preventing or reducing acetaldehyde accumulation in a subject, comprising administering to the subject an effective amount of a composition comprising a compound disclosed herein, wherein after administration, the level of acetaldehyde decreases in at least one cell, at least one cell of the subject or in the subject. In one aspect, such modulation of acetaldehyde dehydrogenase prevents, treats, or reduces alcohol toxicity. In another aspect, such modulation of acetaldehyde dehydrogenase prevents or treats cancer. For example, in a further aspect, the cancer that is prevented or treated can be esophageal squamous cell (ESC) cancer. In an aspect, the composition is a pharmaceutical composition, a dietary supplement, a nutraceutical, further comprises a pharmaceutically acceptable carrier, or a medicament. In an aspect, the composition is administered in a therapeutically effective amount. In an aspect, the composition is administered in a prophylactically effective amount. In an aspect, the composition is administered to a subject who consumes alcohol, ethanol or who has an undesired amount of aldehyde in its body, for example, acetaldehyde. In an aspect, a composition is administered before a subject consumes alcohol, ethanol or who has an undesired amount of aldehyde in its body, for example, acetaldehyde. In an aspect, a composition is administered immediately before a subject consumes alcohol, ethanol or an undesired amount of aldehyde is reached in its body, for example, acetaldehyde. In an aspect, a composition is administered concurrently with a subject consuming alcohol, ethanol or an undesired amount of aldehyde is reached in its body, for example, acetaldehyde. In an aspect, a composition is administered after a subject has consumed alcohol, ethanol or an undesired amount of aldehyde has been reached in its body, for example, acetaldehyde. In an aspect, reducing the level of acetaldehyde in a subject comprises increasing glutathione levels in the subject in many tissues, for example, in the liver. In an aspect, a composition comprises a compound that is a phase 2 inducer. In an aspect, a composition comprises one or more compounds, wherein a compound is sulforaphane, a derivative of sulforaphane, an analog of sulforaphane, an isothiocyanate (ITC), a glucosinolate, erucin, iberin, benzyl-ITC, phenethyl-ITC, propyl-ITC, hexyl-ITC, 4RB-ITC (4-rhamno benzyl-ITC), withaferin A, a steroid, a triterpenoid, TP-225, celastrol, tricyclic (cyano enones), TBE-31, bis(benzylidenes), HBB-2, HBB-4, flavonoids, BNF (Beta naptho flavonoid,) pinostrobin, tectochrisin, kaempferide, a stilbenoid, resveratrol, a sesquiterpene, and zerumbone. In an aspect, acetaldehyde dehydrogenase activity is increased. In an aspect, the amount of acetaldehyde dehydrogenase is increased. In an aspect, co-factors, such as NAD(P)H are modulated, such as increased or decreased. In an aspect, expression of acetaldehyde dehydrogenase is encoded by one or more of the genes *Aldh1a1, Aldh2, Aldh3a1* or a combination thereof. In an aspect, *Aldh2* has one or more polymorphisms. In an aspect, acetaldehyde dehydrogenase is one or more of the enzymes ALDH2, ALDH1A, ALDH3A1, ALDH1B1 or a combination thereof. In an aspect, ALDH2 is mutated (ALDH2*2), wherein a mutation may comprise an insertion, deletion, inversion or other known mutations of the DNA or protein sequence. In an aspect, acetaldehyde dehydrogenase is located in the cytosolic fraction of a cell, microsomal fraction of a cell, mitochondria or in some or all locations. In an aspect, expression of acetaldehyde dehydrogenase increases in a tissue of a subject, for example, in liver, the forestomach, the glandular stomach, the small intestine or other tissues. In an aspect, modulating the expression of acetaldehyde dehydrogenase and/or modulating the activity of acetaldehyde dehydrogenase reduces, lowers, and/or modulates alcohol toxicity. In an aspect, modulating the expression of acetaldehyde dehydrogenase and/or modulating the activity of acetaldehyde dehydrogenase reduces the risk of cancer due to acetaldehyde exposure, for example, esophageal cancer.

Disclosed herein are methods for modulating at least one member of the acetaldehyde dehydrogenase superfamily, wherein a member of the acetaldehyde dehydrogenase superfamily is any enzyme that possesses acetaldehyde dehydrogenase function. That is, any enzyme that catalyzes the conversion of acetaldehyde into acetic acid.

In one aspect, increasing the gene expression of at least one member of the acetaldehyde dehydrogenase superfamily in a subject can comprise administering an effective amount of a phase 2 inducer, thereby increasing the gene expression of at least one member of the acetaldehyde dehydrogenase superfamily. In another aspect, increasing the amount of at least one member of the acetaldehyde dehydrogenase superfamily in a subject can comprise administering an effective amount of a phase 2 inducer, thereby increasing the amount of at least one member of the acetaldehyde dehydrogenase superfamily. In another aspect, increasing the amount of at least one member of the acetaldehyde dehydrogenase superfamily can comprise administering an effective amount of a compound that induces NQO1, thereby increasing the amount of at least one member of the acetaldehyde dehydrogenase superfamily. In another aspect, increasing the amount of at least one member of the acetaldehyde dehydrogenase superfamily can comprise administering an effective amount of a compound that upregulates at least one step in the Keap1/Nrf2/ARE transcription pathway, thereby increasing the amount of at least one member of the acetaldehyde dehydrogenase superfamily. In a further aspect, the compositions may comprise a pharmaceutical composition, a nutraceutical composition, a natural product composition, a medical food, a nutritional supplement, or a composition comprising excipients, diluents, enzymes, cofactors, and delivery vehicle additives.

In one aspect, a phase 2 inducer used to modulate at least one member of the acetaldehyde dehydrogenase superfamily can be sulforaphane or a derivative or an analog, or a combination thereof.

ln the disclosed methods, benefit to the subject may be derived from increasing expression, amount, or activity of acetaldehyde dehydrogenases or members of the acetaldehyde dehydrogenase superfamily. For example, a method disclosed herein may increase expression, amount, or activity or ALDH1A, ALDH2, ALDH3A1, ALDH1B1 or a combination thereof. Additionally, in one aspect, ALDH2 may be mutated. Acetaldehyde dehydrogenase activity may be the result of acetaldehyde dehydrogenase enzymes localized in the cytosol, microsomal fraction, or mitochondria of a cell or a combination thereof.

Acetaldehyde dehydrogenases can be expressed in various cells and tissues throughout a subject. For example, in one aspect, acetaldehyde dehydrogenases can be expressed in the stomach, forestomach, glandular stomach, intestines, or liver. In one aspect, the disclosed methods can increase expression, amount, or activity of acetaldehyde dehydrogenases in any of these tissues or a combination thereof.

In one aspect, expression of acetaldehyde dehydrogenase is encoded by one or more of the genes *Aldh1a1, Aldh2, Aldh3a1* or a combination thereof. In a further aspect, *Aldh2* may have one or more polymorphisms.

The present invention comprises compositions comprising compounds to modulate, reduce, prevent, or treat acetaldehyde accumulation in a subject or reduce, prevent or treat alcohol toxicity by modulating the expression, amount, or activity of enzymes, particularly ALDHs, present in at least one cell of a subject.

In one aspect, the disclosed composition may comprise a phase 2 inducer, various examples of which are disclosed herein. Phase 2 inducers may be involved in activation of Nrf2 regulation, and may be involved in up-regulation of glutathione levels and synthesis. Compounds that are effective in the present invention may comprise compounds that comprise a sulfhydryl group that attacks a cysteine group of an amino acid in a peptide or protein, such as those found in regulatory pathways of a subject directed to responding to toxic events affecting cells. Such a regulatory pathway may include the gene products of Nrf2 regulation, though other regulatory pathways are contemplated by the present document.

Glucosinolates are a class of organic compounds, which may be found in a variety of plants, including cruciferous vegetables. Glucosinolates can serve as precursors for isothiocyanates and phase 2 inducers, including sulforaphane. As used herein, glucosinolate includes the glucosinolate compound and modified glucosinolate compounds, including modifications of the glucose moiety and other structures of the compound.

The present document comprises methods and compositions comprising glucosinolates for reducing the level of acetaldehyde in a subject and treating, reducing or preventing ethanol toxicity due to acetaldehyde accumulation in a subject, particularly one who consumes ethanol. Though not wishing to be bound by any particular theory, it is currently believed that a benefit of crucifer plants is their content of isothiocyanates and their precursor molecules, glucosinolates. Glucosinolates are converted to isothiocyanates by the enzymes such as thioglucosides, for example, myrosinase. Generally, in plant cells, myrosinase and glucosinolates are separated in the cell. However, if the cell is damaged, such as by insect predation, the loss of compartmentalization allows myrosinase or other similarly acting enzymes to come into contact with glucosinolates, which are then converted to isothiocyanates. Myrosinase, EC 3.2.1.147, CAS number 9025-38-1 is known to those of skill in the art, as are similarly acting enzymes that convert precursor molecules, such as glucosinolates, to more active compounds, such as isothiocyanates, for instance, sulforaphane.

The present document comprises methods and compositions comprising one or more enzymes, and/or one or more types of enzymes, and optionally co-factors or other enzymes in the metabolic pathway, for reducing the level of acetaldehyde in a subject and treating, reducing or preventing ethanol toxicity due to acetaldehyde accumulation in a subject who consumes ethanol. Enzymes contemplated by the present document, referred to herein as enzymes of the present document, comprise, but are not limited to, myrosinase, thioglucosidases, glutathione transferases, NAD(P)H:quinone reductase (QR) and glucuronosyltransferases, which have similar activities or are in related pathways. For example, as known in the art, in the presence of water, myrosinase cleaves the glucose group from a glucosinolate. The remaining molecule then converts to a thiocyanate, an isothiocyanate or a nitrile; these are the active substances that serve as a defense for the plant. The hydrolysis of glucosinolates by myrosinase or other enzymes of the present document or similarly acting enzymes can yield a variety of products, depending on various physiological conditions such as pH and the presence of certain cofactors. Reactions have been observed to share the initial steps. First, the β-thiogtucoside linkage is cleaved by myrosinase, releasing D-glucose. The resulting aglycone undergoes a spontaneous Lossen-like rearrangement, releasing a sulfate. The last step in the mechanism is subject to the greatest variety depending on the physiological conditions under which the reaction takes place. At neutral pH, the primary product is the isothiocyanate. Under acidic conditions (pH < 3), and in the presence of ferrous ions or epithospecifier proteins, the formation of nitriles is favored instead.

Methods for the extraction of natural products as sources for compounds such as sulforaphane, include methods for extraction from plant sources in contrast to those produced by chemical synthetic methods. Extraction from plant sources, such as cruciferous vegetables, include, but are not limited to, homogenization of the vegetables in cold water, lyophilization, extraction of the resultant powder with acetonitrile, filtration and evaporative concentration. Other methods for extraction of compounds from plants are known in the art and are contemplated by the present invention, and may comprise, for example, extractions of seeds and sprouts to produce compounds of the present invention, such as taught by U.S. Patent No. 5,725,895. Known methods for extracting natural products, particularly from cruciferous plants, comprise extraction methods comprising boiling water extraction of desired compounds.

Though not wishing to be bound by any particular theory, it is currently believed that glucosinolates are a precursor molecule without activity that is then converted by enzymes into an active form, for example an isothiocyanate such as sulforaphane (which may be referred to herein as a more active compound because the compound is more active in particular assays compared to the activity of its precursor molecule). Compositions of the present document comprise one or more precursor compounds, such as glucosinolates, and/or may comprise more active molecules such as products made by enzymatic activity on glucosinolates, for example sulforaphane, or both one or more precursor compounds and one or more active compounds, and further may optionally comprise one or more enzymes and/or co-factors of such enzymes that use the precursor compound or the more active compound as a substrate. Glucosinolates in foods are converted at least partially to isothiocyanates in humans, by, it is currently believed, microorganisms of the gut. For example, a composition of the present document may comprise one or more precursor compounds, such as glucosinolate, for reducing the level of acetaldehyde in a subject and treating, reducing or preventing ethanol toxicity due to acetaldehyde accumulation in a subject who consumes ethanol. A composition may further comprise one or more enzymes for which the compound provided in the composition is a substrate molecule of the one or more enzymes. The composition may be provided in a unitary delivery vehicle or may be provided in two or more delivery vehicles which may be provided simultaneously, sequentially, or in other administrative methods.

### CRUCIFEROUS PLANT SOURCES OF ALDH MODULATORS

Plant sources suitable for use in the methods and compositions disclosed herein may be any portion of a cruciferous plant, including, but not limited to cells, seeds, sprouts, leaves, stalks, roots, flowers and other plant structures. Plant sources contemplated by the present invention comprise, but are not limited to, plants from the family Cruciferae, such as Brassiceae, and including Brassicinae. For example, the plant source may be Brassica oleracea selected from the group of varieties of acephala (kale, collards, wild cabbage, curly kale), medullosa (marrowstem kale), ramosa (thousand head kale), alboglabra (Chinese kale), botrytis (cauliflower, sprouting broccoli), costata (Portuguese kale), gemmifera (Brussels sprouts), gongylodes (kohlrabi), italica (broccoli), palmifolia (Jersey kale), sabauda (savoy cabbage), sabellica (collards), and selensia (borecole), among others.

Useful broccoli cultivars to be used in the method and compositions disclosed herein are Saga, DeCicco, Everest, Emerald City, Packman, Corvet, Dandy Early, Emperor, Mariner, Green Comet, Green Valiant, Arcadia, Calabrese Caravel, Chancellor, Citation, Cruiser, Early Purple Sprouting Red Arrow, Eureka, Excelsior, Galleon, Ginga, Goliath, Green Duke, Greenbelt, Italian Sprouting, Late Purple Sprouting, Late Winter Sprouting White Star, Legend, Leprechaun, Marathon, Mariner, Minaret (Romanesco), Paragon, Patriot, Premium Crop, Rapine (Spring Raab), Rosalind, Salade (Fall Raab), Samurai, Shogun, Sprinter, Sultan, Taiko, and Trixie. However, many other broccoli cultivars are suitable.

Useful cauliflower cultivars to be used in the method and compositions disclosed herein are Alverda, Amazing, Andes, Burgundy Queen, Candid Charm, Cashmere, Christmas White, Dominant, Elby, Extra Early Snowball, Fremont, Incline, Milkyway Minuteman, Rushmore, S-207, Serrano, Sierra Nevada, Siria, Snow Crown, Snow Flake, Snow Grace, Snowbred, Solide, Taipan, Violet Queen, White Baron, White Bishop, White Contessa, White Corona, White Dove, White Flash, White Fox, White Knight, White Light, White Queen, White Rock, White Sails, White Summer, White Top, Yukon. However, many other cauliflower cultivars are suitable.

A composition of the present invention comprises sulforaphane, an organosulfur compound (1-isothiocyanato-4R-(methylsulfinyl)butane); derivatives known in the art, such as dithiocarbamate derivatives and others disclosed herein; and analogs known in the art and/or disclosed herein. Sulforaphane is a hormetic drug that, though not wishing to be bound by any particular theory, is thought to induce a general "cell protective" response. Sulforaphane is an active component of many plants and for example, may be extracted from broccoli sprouts, or may be made by chemical synthetic methods. Sulforaphane may be obtained from may be obtained synthetically or from natural sources, such as from lyophilized, freeze dried extracts of 3-day-old or older broccoli sprouts. Broccoli sprouts are widely consumed all over the world by a very large number of individuals, without any reports of adverse effects. Human research studies have also not shown any significant adverse effects by administration of sulforaphane.

Though not wishing to be bound by any particular theory, it is believed that sulphoraphane may provide protection against oxidative and inflammatory stress, such as disturbances of systems that protect cells against oxidative damage, heat shock, and disturbances caused by protein misfolding. These protective mechanisms are thought to be mediated by the transcription factor Nrf2 which controls expression of genes of the human genome via the Keap1/Nrf2/ARE regulatory system. This system may be upregulated in many tissues including the liver and GI tract by sulforaphane.

Sulforaphane may be derived from synthetic or natural sources. Plant sources for sulforaphane include, but are not limited to, cruciferous plants. Plant sources suitable for use in the methods and compositions disclosed herein may be any portion of a cruciferous plant, including, but not limited to cells, seeds, sprouts, leaves, stalks, roots, flowers and other plant structures. Plant sources contemplated by the present invention comprise, but are not limited to, plants from the family Cruciferae, such as Brassiceae, and including Brassicinae. For example, the plant source may be Brassica oleracea selected from the group of varieties of acephala (kale, collards, wild cabbage, curly kale), medullosa (marrowstem kale), ramosa (thousand head kale), alboglabra (Chinese kale), botrytis (cauliflower, sprouting broccoli), costata (Portuguese kale), gemmifera (Brussels sprouts), gongylodes (kohlrabi), italica (broccoli), palmifolia (Jersey kale), sabauda (savoy cabbage), sabellica (collards), and selensia (borecole), among others.

Furthermore, the art is familiar with sulforaphane analogs, which include, but are not limited to, the following: 6-isothiocyanato-2-hexanone, exo-2-acetyl-6-isothiocyanatonorbornane, exo-2-isothiocyanato-6-methylsulfonylnorbornane, 6-isothiocyanato-2-hexanol, 1-isothiocyanato-4-dimethylphosphonylbutane, exo-2-(1'-hydroxyethyl)-5-isothiocyanatonorbornane, exo-2-acetyl-5-isothiocyanatonorbornane, 1-isothiocyanato-5-methylsulfonylpentane, cis-3-(methylsulfonyl)cyclohexylmethylisothiocyanante and trans-3-(methylsulfonyl)cyclohexylmethylisothiocyanante.

A composition of the present invention may comprise the compound sulforaphane, an organosulfur compound (1-isothiocyanato-4R-(methylsulfinyl)butane).. Sulforaphane is an active component of many plants and for example, may be extracted from broccoli sprouts, or may be made by chemical synthetic methods. Sulforaphane may be obtained from lyophilized, freeze dried extracts of 3 -day-old broccoli sprouts. In one aspect, the composition comprising sulforaphane is used for the treatment of alcohol toxicity. In another aspect, the composition comprising sulforaphane is used for reducing or preventing acetaldehyde accumulation.

In one aspect, a disclosed composition comprises compounds derived from natural or synthetic sources which may be used in a composition to treat acetaldehyde accumulation, which may result from ethanol consumption. In another aspect, a disclosed composition is to treat alcohol toxicity.

In one aspect, a disclosed composition comprising one or more enzymes for which the compound provided in the composition is a substrate molecule of the one or more enzymes. In a further aspect, the composition can comprise enzyme co-factors, substrates, metals, enzymes, co-enzymes or other components of the enzymatic pathway. In a further aspect, the composition may be provided in a unitary delivery vehicle or may be provided in two or more delivery vehicles which may be provided simultaneously, sequentially, or in other administrative methods.

In one aspect, a disclosed composition is in a form selected from the group consisting of tablet form, liquid form, powder form, capsule form, injectable form and spray form. In another aspect, the composition may be provided in a unitary delivery vehicle or may be provided in two or more delivery vehicles which may be provided simultaneously, sequentially, or in other administrative methods.

A composition of the present document may comprise one or more compounds disclosed herein, for example, a compound that increases expression, amount, or activity of at least one acetaldehyde dehydrogenase, a compound that reduces or prevents toxicity associated with consumption of ethanol or toxic aldehydes, a compound that increases the amount of acetaldehyde dehydrogenase in a cell. This can include, for example, glucosinolates, sulforaphane and/or its derivatives and analogs. In an aspect, a composition comprises sulforaphane. In another aspect, a composition comprises derivatives of sulforaphane. In an aspect, a composition comprises analogs of sulforaphane. In an aspect, a composition comprises an isothiocyanate (ITC), including but not limited to, sulforaphane, erucin, iberin, benzyl-ITC, phenethyl-ITC, propyl-ITC, hexyl-ITC, and 4RB-ITC. In an aspect, a composition comprises a steroid, including but not limited to, withaferin A. In an aspect, a composition comprises a triterpenoid, including but not limited to, TP-225 and celastrol. In an aspect, a composition comprises tricyclic (cyano enones), including but not limited to, TBE-31. In an aspect, a composition comprises Bis(benzylidenes), including but not limited to, HBB-2 and HBB-4. In an aspect, a composition comprises a flavonoid, including but not limited to, BNF, pinostrobin, tectochrisin, and kaempferide. In an aspect, a composition comprises a stilbenoid, including but not limited to, resveratrol. In an aspect, a composition comprises a sesquiterpene, including but not limited to, zerumbone.

In one aspect, a disclosed composition comprises a combination of compounds disclosed herein, for example a composition comprising one or more of an isothiocyanate, a glucosinolate, sulforaphane, a sulforaphane derivative, a sulforaphane analog, erucin, iberin, benzyl-ITC, phenethyl-ITC, propyl-ITC, hexyl-ITC, 4RB-ITC, withaferin A, a steroid, a triterpenoid, TP-225, celastrol, tricyclic (cyano enones), TBE-31, bis(benzylidenes), HBB-2, HBB-4, a flavonoid, BNF, pinostrobin, tectochrisin, kaempferide, a stilbenoid, resveratrol, a sesquiterpene, and/or zerumbone.

In one aspect, a disclosed composition of the present invention may comprise a compound that is present as a structure represented by a formula shown below, or a subgroup or pharmaceutically acceptable salts thereof. 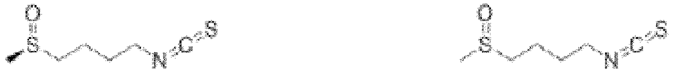 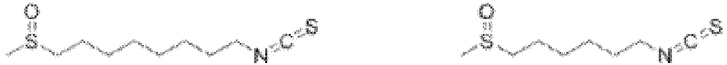 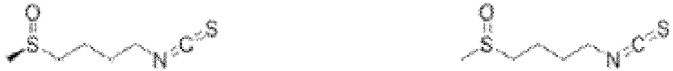 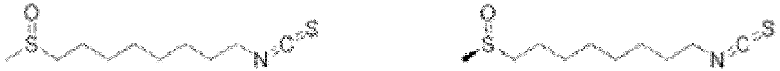 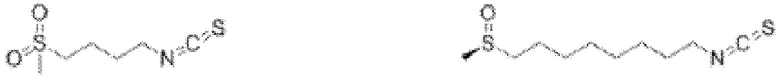 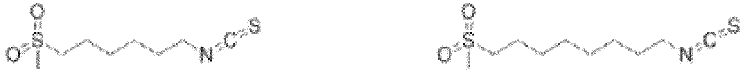 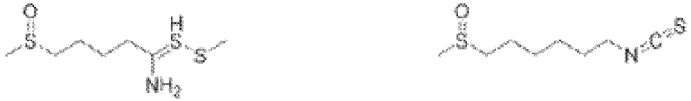 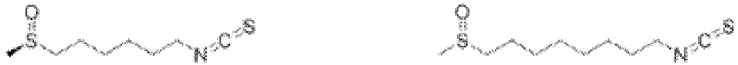 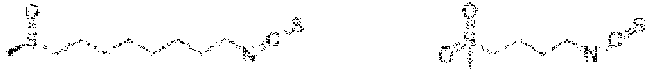 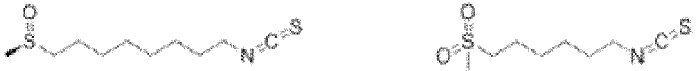 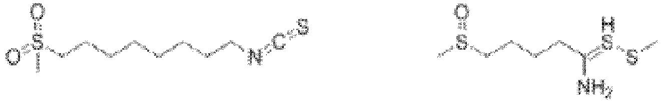 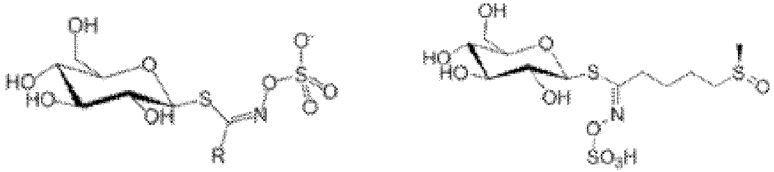 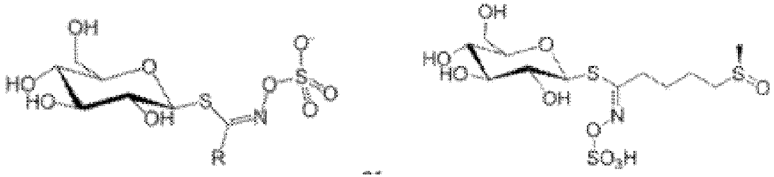 or

In an aspect, a composition comprises medical foods used to treat acetaldehyde accumulation, which may result from ethanol consumption. In another aspect, a composition comprises a dietary or nutritional supplements used to treat acetaldehyde accumulation, which may result from ethanol consumption. In another aspect, a composition comprises a pharmaceutical or nutraceutical composition.

In an aspect, a disclosed composition comprises excipients, diluents, enzymes, cofactors, pharmaceutically acceptable carriers and delivery vehicle additives.

In an aspect, the compound that modulates the expression, amount, or activity of acetaldehyde dehydrogenase is a phase 2 inducer. For example, in one aspect, the compound can be sulforaphane.

In an aspect, a composition used in the disclosed methods can be formulated in a form appropriate for any means of administration. Composition forms include, but are not limited to, tablet form, liquid form, powder form, capsule form, injectable form and spray form. The form of the composition can dictate the route of administration, examples of which have been disclosed herein and are known in the art. An ordinary person of skill in the art, for example, a physician, would know the type of formulation appropriate for implementing the disclosed methods and the appropriate route of administration. In another aspect, the compositions of the disclosed methods may further comprise a pharmaceutically acceptable carrier when necessary. The carrier may differ depending on the route of administration and may include excipients, fillers, antioxidants, bacteriostats, buffers solutions, and other formulation components known in the art.

In one aspect, a composition of the disclosed methods may comprise a pharmaceutical composition, a nutraceutical composition, a natural product composition, a medical food, a medicament, a nutritional supplement, or a composition comprising excipients, diluents, enzymes, cofactors, pharmaceutically acceptable carriers, and delivery vehicle additives.

In an aspect, the compositions are administered in a therapeutically effective amount, thereby reducing the level of acetaldehyde in a subject or increases the activity or expression of acetaldehyde dehydrogenase to an extent that the toxicities associated with acetaldehyde dehydrogenase accumulation are alleviated.

In an aspect, the compositions are administered in a prophylactically effective amount, thereby preventing an increase in the level of acetaldehyde in a subject or increasing the activity or expression of acetaldehyde dehydrogenase to an extent that the toxicities associated with acetaldehyde dehydrogenase accumulation are prevented.

In an aspect, the subject may be in need of a reduction in the level of acetaldehyde in the subject or an increase in the activity or expression of acetaldehyde dehydrogenase due to the consumption of ethanol. In another aspect, the subject consuming alcohol may express non-mutated ALDHs at normal physiological levels. In another aspect, the subject consuming alcohol may express non-mutated ALDHs at lower than physiological levels. In another aspect, the subject consuming alcohol may express non-mutated ALDHs at higher than physiological levels. In another aspect, the subject consuming alcohol may express mutated ALDHs at normal physiological levels. In another aspect, the subject consuming alcohol may express mutated ALDHs at lower than physiological levels. In another aspect, the subject consuming alcohol may express mutated ALDHs at higher than physiological levels.

In an aspect, a disclosed composition may be administered to a subject before the subject consumes ethanol. In this context, before can mean for a period of time ranging from days, week, months, or years or any time in between and may include chronic administration on a routine basis. A routine basis could be in the form of daily administration, similar to a multi-vitamin. In another aspect, the compositions disclosed herein may be administers to the subject immediately before the subject consumes ethanol. Immediately before can mean the day before ethanol consumption commences or up to the seconds before it commences or any time in between.

In another aspect, the disclosed compositions may be administered to a subject concurrently while the subject consumes ethanol. That is, once the subject commences drinking, the subject may simultaneously commence administration of the disclosed compositions. Concurrent administration can be one administration as ethanol consumption begins or continuous or periodic administration throughout the entire time frame that ethanol consumption occurs.

In another aspect, the disclosed compositions may be administered to a subject after the subject consumes ethanol. For example, administration of the compositions could begin following the initial commencement of ethanol consumption or following the completion of ethanol consumption. As before, this administration may be chronic, continuous, or periodic. Administration of the disclosed compositions may occur over a period of hours, days, week, months or years or any time in between after the consumption of ethanol.

In an aspect, methods of administering an effective amount of disclosed compositions may also result in an increase in glutathione levels in many tissues, for example, in the liver. This is a positive off-target effect that can occur as a result of stimulating NQO1 and/or the Keap1/Nrf2/ARE pathway with certain phase 2 inducers. Increasing glutathione levels also provides benefit in that it may facilitate the clear of additional toxic aldehydes.

The present document comprises a method and assay for determining the effectiveness of a compound for treating acetaldehyde accumulation, comprising contacting a first cell with a compound to be tested, comparing the response of the first cell with the response of an identical second cell, and determining whether or not the tested compound increases the amount, the expression of, or the enzymatic activity of at least one member of the acetaldehyde dehydrogenase superfamily in the first cell when compared to the second cell. The assay may further comprise comparing the response of the first cell to a cell that has been contacted with sulforaphane. The assay may further comprise comparing the response of the first cell to a cell that has been contacted with a compound that is a phase 2 inducer. The assay may further comprise comparing the response of the first cell to a cell that has been contacted with a compound that upregulates at least one step in the Keap1/Nrf2/ARE transcription pathway. The assay may further comprise comparing the response of the first cell to a cell that has been contacted with a compound that induces NQO1. Cells may or may not be treated with ethanol, and such treatment may be prior to, concurrent with or after contacting the cells with a test or known compound. Increase in the amount, the expression of, or the enzymatic activity of at least one member of the acetaldehyde dehydrogenase superfamily may be measured by assays and components that are known for those purposes by those of skill in the art.

It is to be understood that the present invention, comprising methods and compositions, is not limited to specific synthetic methods unless otherwise specified, or to particular reagents unless otherwise specified, and as such may, of course, vary. It is also to be understood that the terminology used herein is for the purpose of describing particular aspects only and is not intended to be limiting. Although any methods and materials similar or equivalent to those described herein can be used in the practice or testing of the present invention, example methods and materials are now described.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, and the number or type of embodiments described in the specification.

The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

### DEFINITIONS

As used in the specification and the appended claims, the singular forms "a," "an" and "the" include plural referents unless the context clearly dictates otherwise. Thus, for example, reference to "a phase 2 inducer," "an amount," or "the subject" includes mixtures of two or more such phase 2 inducers, amounts, or subjects, and the like.

Ranges can be expressed herein as from "about" one particular value, and/or to "about" another particular value. When such a range is expressed, a further aspect includes from the one particular value and/or to the other particular value. Similarly, when values are expressed as approximations, by use of the antecedent "about," it will be understood that the particular value forms a further aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units is also disclosed. For example, if 10 and 15 are disclosed, then 11, 12, 13, and 14 are also disclosed.

As used herein, the terms "optional" or "optionally" mean that the subsequently described event or circumstance may or may not occur and that the description includes instances where said event or circumstance occurs and instances where it does not.

As used herein, the term "subject" refers to the target of administration, e.g., an animal. Thus the subject of the herein disclosed methods can be a vertebrate, such as a mammal, a fish, a bird, a reptile, or an amphibian. The term "subject" also includes domesticated animals (e.g., cats, dogs, etc.), livestock (e.g., cattle, horses, pigs, sheep, goats, fish, bird, etc.), and laboratory animals (e.g., mouse, rabbit, rat, guinea pig, fruit fly, etc.). In one aspect, the subject is a mammal. In one aspect, the subject is a human. The term does not denote a particular age or sex. Thus, adult, child, adolescent and newborn subjects, as well as fetuses, whether male or female, are intended to be covered.

The term "patient" refers to a subject afflicted with a toxicity, disease or disorder. As used herein, the term "recipient" refers to a subject who receives the methods or compositions disclosed herein.

As used herein, the term "treatment" refers to the medical management of a patient or subject with the intent to cure, ameliorate, stabilize, or prevent a toxicity, disease, pathological condition, or disorder. This term includes the use for aesthetic and self-improvement purposes; for example, such uses include, but are not limited to, the use of the claimed methods for improving nutrition, metabolic condition, energy level, or sense of well-being. This term includes active treatment, that is, treatment directed specifically toward the improvement of a toxicity, disease, pathological condition, or disorder and also includes causal treatment, that is, treatment directed toward removal of the cause of the associated toxicity, disease, pathological condition, or disorder. In addition, this term includes palliative treatment, that is, treatment designed for the relief of symptoms rather than the curing of the toxicity, disease, pathological condition, or disorder; preventative treatment, that is, treatment directed to minimizing or partially or completely inhibiting the development of the associated toxicity, disease, pathological condition, associated symptoms or disorder; and supportive treatment, that is, treatment employed to supplement another specific therapy directed toward the improvement of the associated toxicity, disease, pathological condition, associated symptoms or disorder. In various aspects, the term covers any treatment of a subject, and includes: (i) preventing the toxicity from occurring in a subject that can be predisposed to the toxicity but has not yet been diagnosed as having a predisposition; (ii) inhibiting the toxicity, i.e., arresting its development; or (iii) relieving the toxicity, i.e., causing regression of the toxicity and its associated symptoms.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the term "diagnosed" means having been subjected to a physical examination by a person of skill, for example, a physician, and found to have a condition that can be diagnosed or treated by the compounds, compositions, or methods disclosed herein. For example, "diagnosed with a mutation in acetaldehyde dehydrogenase" means having been subjected to a physical examination by a person of skill, for example, a physician, and found to have a condition that can be diagnosed or treated by a compound or composition that can promote acetaldehyde dehydrogenase function and/or acetaldehyde dehydrogenase expression. As a further example, "diagnosed with a need for increasing acetaldehyde dehydrogenase" refers to having been subjected to a physical examination by a person of skill, for example, a physician, and found to have a condition characterized by sub-optimal levels of or mutations of acetaldehyde dehydrogenase and therefore the compositions and methods disclosed herein would be beneficial to the subject. Such a diagnosis can be in reference to a disorder, such as acetaldehyde dehydrogenase mutations, and the like, as discussed herein.

As used herein, the terms "administering" and "administration" refer to any method of providing a pharmaceutical or nutraceutical preparation to a subject. Such methods are well known to those skilled in the art and include, but are not limited to, oral administration, transdermal administration, intradermal administration, administration by inhalation, nasal administration, topical administration, intravaginal administration, intraurethral administration, ophthalmic administration, intraaural administration, intracerebral administration, intrathecal administration, rectal administration, sublingual administration, buccal administration, intraparetoneal and parenteral administration, and includes injectables such as intravenous administration, intra-arterial administration, intramuscular administration, and subcutaneous administration. Administration can be continuous or intermittent. In various aspects, a preparation can be administered therapeutically, that is, administered to treat an existing disease or toxicity. In further aspects, a preparation can be administered prophylactically, that is, administered for prevention of a disease or toxicity.

As used herein, the terms "effective amount" and "amount effective" refer to an amount that is sufficient to achieve the desired result or to have an effect on an undesired condition. For example, a "therapeutically effective amount" refers to an amount that is sufficient to achieve the desired therapeutic result or to have an effect on undesired symptoms but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of a compound at levels lower than those required to achieve the desired therapeutic effect and to gradually increase the dosage until the desired effect is achieved.

If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. Dosage can vary and can be administered in one or more dose administrations daily, for one or several days, weeks, months, or years, or any time in between. Guidance can be found in the literature for appropriate dosages for given classes of pharmaceutical products.

In further aspects, a preparation can be administered in a "prophylactically effective amount", that is, an amount effective for prevention of a toxicity, disease or condition.

The term "pharmaceutically acceptable" describes a material that is not biologically or otherwise undesirable, i.e., without causing an unacceptable level of undesirable biological effects or interacting in a deleterious manner.

As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose.

As used herein, the term "derivative" refers to a compound having a structure derived from the structure of a parent compound (e.g., a compound disclosed herein) and whose structure is sufficiently similar to those disclosed herein and based upon that similarity, would be expected by one skilled in the art to exhibit the same or similar activities and utilities as the claimed compounds, or to induce, as a precursor, the same or similar activities and utilities as the claimed compounds. Exemplary derivatives include salts, esters, amides, salts of esters or amides, and N-oxides of a parent compound.

As used herein, the term "dietary supplement" refers to a compound or composition that is intended to compensate the dietary intake of a subject with regard to specific nutrients, vitamins, minerals, or other dietary components that may be missing or deficient in the subject's normal diet. Additionally, a dietary supplement may be a foodstuff or a composition derived thereof that is intended to compensate for pathological or physiological deficiencies of a subject.

As used herein, the term "acetaldehyde dehydrogenase superfamily" refers to any dehydrogenase enzymes that catalyze aldehydes, such as the conversion of acetaldehyde into acetic acid. There are at least seventeen known genes that encode enzymes with this activity including, but not limited to, *Aldh1a1, Aldh3a1, Aldh2* and *Aldh1B1,* or *Aldh5.* Enzymes within this family include, but are not limited to, ALDH1 and ALDH2; however, this term is meant to include all enzymes with the stated activity and all enzymes encoded by the associated genes including those that contain inherited, induced, or spontaneous polymorphisms in their nucleotide or amino acid sequences; inherited, induced, or spontaneous mutants; isozymes; and splice variants.

As used herein, the terms "phase 2 inducer" refers to compositions that increase the expression, amount, or activity of enzymes involved in phase 2 metabolism. Phase 2 metabolism is often the second metabolic step before excretion of a compound. Phase 2 metabolism consists primarily of conjugation reactions including, but not limited to, methylation, sulphation, acetylation, glucuronidation, glutathione conjugation, and glycine conjugation. Examples of enzymes that participate in phase 2 metabolism include, but are not limited to, methyltransferases, sulfotransferases, acetyl Co-enzyme A, Glutathione-S-transferase, N-acetyltransferases, and UDP glucuronosyltransferases. Examples of compositions that are known phase 2 inducers include, but are not limited to, an isothiocyanate, a glucosinolate, sulforaphane, a sulforaphane derivative, a sulforaphane analog, erucin, iberin, benzyl-ITC, phenethyl-ITC, propyl-ITC, hexyl-ITC, 4RB-ITC, withaferin A, a steroid, a triterpenoid, TP-225, celastrol, tricyclic (cyano enones), TBE-31, bis(benzylidenes), HBB-2, HBB-4, a flavonoid, BNF, pinostrobin, tectochrisin, kaempferide, a stilbenoid, resveratrol, a sesquiterpene, and zerumbone.

As used herein, the term "alcohol" refers to alcoholic beverages that contain ethanol, for example, beer, wine, or spirits. For the purposes of this disclosure, the terms alcohol and ethanol may be used interchangeably.

### REFERENCES

Abdullah, A., Kitteringham. N. R., Jenkins, R. E., Goldring, C, Higgins, L., Yamamoto, M., Hayes, J., and Park, B. K. (2012) Pharmacol. Rep. 64, 680-697.
Alnouti, Y., and Klaassen, C. D. (2008) Toxicol. Sci. 101, 51-64 Brooks P. J., Enoch MA., Goldman, D., Li, TK., and Yokoyama, A. (2009) PLoS Med. 6, e50
Budas, G. R., Churchill, E. N., Disatnik, M. FL, Sun, L., and Mochly-Rosen, D. (2010) Cardiovasc. Res. 88, 83-92
Chen, C. FL, Budas, G. R., Churchill, E. N., Disatnik, M. FL, Hurley, T. D., and Mochly-Rosen, D. (2008) Science. 321, 1493-1495
Cho, H. Y, Reddy, S. P., Debiase, A., Yamamoto, M., and Kleeberger, S. R. (2005) Free. Radic. Biol. Med. 38, 325-343
Churchill, E. N., Disatnik, M. H., and Mochly-Rosen, D. (2009) J. Mol. Cell. Cardiol. 46, 278-284
Crabb, D. W., Matsumoto, M., Chang, D., and You M. (2004) Proc. Nutr. Soc. 63, 49-63
Dinkova-Kostava, A.T., Talalay, P., Sharkey, J., Zhang, U., Holtzclaw, W.D., Wang, X.J., David, E., Schiavoni, K.H., Finlayson, S., Mierke, D.F., and Honda, T. (2010) J. Biol. Chem. 285, 33747-33755
Elbarbry, F., and Elrody, N. (2011) J. Med. Plants Res. 5, 473-484
Eng, M. Y., Luczak, S. E., and Wall, T. L. (2007) Alcohol. Res. Health. 30, 22-27
Fahey, J. W., Dinkova-Kostova, A. T., Stephenson, K. K., and Talalay, P. (2004) Methods Enzymol. 382, 243-258
Gong, D., Zhang, H., and Hu, S. (2012) J. Mol. Cell Cardiol. [Epub ahead of print]
Guerrero-Beltrán, C. E., Calderon-Oliver, M., Pedraza-Chaverri, J., and Chirino, Y.I. (2010) Exp. Toxicol. Pathol. 64, 503-508
Hald, J., and Jacobsen, E. (1948) Lancet. 2, 1001-1004
Harada, S., Agarwal, D. P., and Goedde, H. W. (1981) Lancet. 31, 982
Haseba, T., Sugimoto, J., Sato, S., Abe, Y., and Ohno, Y. (2008) Metabolism. 57, 1753-1759
Healy, Z. R., Liu, H., Holtzclaw, W. D., and Talalay, P. (2011) Cancer. Epidemiol. Biomarkers. Prev. 20, 1516-1523
Higdon, J. V., Delage, B., Williams, D. E., and Dashwood, R. H. (2007) Pharmacol. Res. 55, 224-236
Hill, B. G., and Bhatnagar, A. (2009) Circ. Res. 105, 1044-1046
Honda, H., Sundarararian, C,Yoshizawa, H.,Su, X.,Honda, Y., Liby, K.T.,Sporn, MB., and Gribble, G.W. (2007) J. Med. Chem. 50, 1731-1734.
Impraim, C, Wang, G., and Yoshida, A. (1982) Am. J. Hum. Genet. 34, 837-841
Kakuda, T., Sakane, I., Takihara, T., Tsukamoto, S., Kanegae, T., and Nagoya, T. (1996) Biosci. Biotechnol. Biochem. 60, 1450-1454
Kano, M., Ishikawa, F., Matsubara, S., Kikuchi-Hayakawa, H., and Shimakawa, Y. (2002) J. Nutr. B2, 238-244
Kitteringham, N. R., Abdullah, A., Walsh, 1, Randle, L., Jenkins, R. E., Sison, R., Goldring, C. E., Powell, H., Sanderson, C, Williams, S., Higgins, L., Yamamoto, M., Hayes, J., and Park, B. K. (2010) J. Proteomics. 73, 1612-1631
Koivula, T., Koivusalo, M., and Lindros, K. O. (1975) Biochem. Pharmacol. 24, 1807-1811
Lee, J. M., Calkins, M. J., Chan, K., Kan, Y. W., and Johnson, J. A. (2003) J. Biol. Chem. 278, 12029-12038
Nair, S., Xu, C, Shen, G., Hebbar, V., Gopalakrishnan, A., Hu, R., Jain, M. R., Lin, W., Keum, Y. S., Liew, C, Chan, J. Y., and Kong, A. N. (2006) Pharm. Res. 23, 2621-2637
Oze, I., Matsuo, K., Wakai, K., Nagata, C, Mizoue, T., Tanaka, K., Tsuji, I., Sasazuki, S., Inoue, M., and Tsugane, S. (2011) Jpn. J. Clin. Oncol. 41, 677-692
Perez-Miller, S., Younus, H., Vanam, R., Chen, C. H., Mochly-Rosen, D., and Hurley, T. D. (2010) Nat. Struct. Mol. Biol. 17, 159-64
Prochaska, H. J., and Santamaria, A. B. (1988) Anal. Biochem. 169, 328-336
Santisteban, I., Povey, S., West, L. F., Parrington, J. M., and Hopkinson, D. A. (1985) Ann. Hum. Genet. 49, 87-100.
Shen, G., Xu, C, Hu, R., Jain, M. R., Nair, S., Lin, W., Yang, C. S., Chan, J. Y., and Kong A. N. (2005) Pharm. Res. 22, 1805-1820.
Sreerama, L., and Sladek, N. E. (2001) Chem. Biol. Interact. 130-132, 247-60
Suganuma, H., Fahey, J. W., Bryan, K. E., Healy, Z. R., and Talalay, P. (2011) Biochem. Biophys. Res. Commun. 405, 146-151
Talalay, P., and Fahey, J. W. (2001) J. Nutr. 131, 3027S-3033S
Talalay, P., Dinkova-Kostova, A. T., and Holtzclaw, W. D. (2003) Adv. Enzyme. Regul. 43, 121-134
Talalay, P., Fahey, J. W., Holtzclaw, W. D., Prestera, T., and Zhang, Y. (1995) Toxicol. Lett. 82-83, 173-179
Tottmar, O., Marchner, H., and Pettersson, H. (1978) Anal. Biochem. 91, 241-249.
Toyama, T., Shinkai, Y., Yasutake, A., Uchida, K., Yamamoto, M., and Kumagai, Y. (2011) Environ. Health Perspect. 119, 1117-1122.
Vasiliou, V., Pappa, A., and Estey, T. (2004) Drug. Metab. Rev. 36, 279-299
Vasiliou, V., Pappa, A., and Petersen, D. R. (2000) Chem. Biol. Interact. 129, 1-19
Wierzchowski, J., Interewicz, E., Wroczynski, P., and Orlanska, I. (1996) Anal. Chim. Acta. 319, 209-219
Wierzchowski, J., Wroczynski, P., Laszuk, K., and Interewicz, E. (1997) Anal Biochem. 245, 69-78
Xue, L., Xu, F., Meng, L., Wei, S., Wang, J., Hao, P., Bian, Y., Zhang, Y., and Chen Y. (2012) FEBS Lett. 586, 137-142
Yamaji, T., Inoue, M., Sasazuki, S., Iwasaki, M., Kurahashi, N., Shimazu, T., and Tsugane, S. (2008) Int. J. Cancer. 15, 1935-1940
Yokoyama, T., Yokoyama, A., Kato, H., Tsujinaka, T., Muto, M., Omori, T., Haneda, T., Kumagai, Y., Igaki, H., Yokoyama, M., Watanabe, H., and Yoshimizu, H. (2003) Cancer Epidemiol. Biomarkers Prev. 12, 1227-1233
Yoshida, A., Huang, I. Y., and Ikawa, M. (1984) Proc. Natl. Acad. Sci. U.S.A. 81, 258-261
Zhang, Y., Talalay, P., Cho, C. G., and Posner, G. H. (1992) Proc. Natl. Acad. Sci. U. S. A. 89,2399-2403.

### EXAMPLES

From the data and experiments shown herein, it is shown that sulforaphane (SF), an example of a natural, potent phase 2 inducer, induces ALDH expression and enzymatic activity in vitro and in vivo. Though not wishing to be bound by any particular theory, it is believed that sulphoraphane, and other phase inducers effect the Keap1/Nrf2/ARE pathway, though other pathways and effects, such as modulations of NAD(P)H-related pathways and enzymes may also be affected. The resulting increases in enzyme amount and/or activity of ALDHs, such as *Aldh1a1* and ALDH2, in liver and other organs enhance the metabolism of acetaldehyde and accelerate the elimination of acetaldehyde from the blood, such as after consumption of ethanol.

In a series of in vitro studies, total ALDH activity was measured in cell homogenates to assess the potencies of SF and other various phase 2 inducers for induction of ALDH. The human genome contains at least 17 genes that are members of ALDH superfamily (Vasillou 2004), so the total ALDH activity measured, as disclosed herein, can include activities of multiple ALDH enzymes. As a result of evaluating 21 phase 2 inducers belonging to over 8 chemical classes, it was observed that the potencies of these compounds for the induction of ALDH were associated with the concomitant induction of NQO1.

Additionally, increase of total ALDH activity by SF was reduced in Nrf2-/- MEFs, which indicated that induction of ALDHs may be regulated by the Keap1/Nrf2/ARE pathway. Up-regulation of the basal and induced expression levels of ALDH1A1 (*Aldh1a1*) and ALDH2 (*Aldh2*) in WT mice were observed in these studies consistently. Herein, it was confirmed that phase 2 inducers with different structures increase gene expression of *Aldh1a1* and *Aldh2* in Hepa1c1c7 cells. Furthermore, intake of SF increased ALDH activity in both cytosol and mitochondria of mice, particularly in the liver, concomitantly with increased gene expressions of *Aldh1a1* and *Aldh2.*

Time course measurement showed that total ALDH activity increased after 12-18 h of treatment with SF, which occurs following a series of responses including transcription, translation and protein synthesis, theorized to be through the Keap1/Nrf2/ARE pathway.

After ingestion, a large portion of ethanol (-80%) is absorbed in the small intestine and a small portion (-20%) is absorbed in the stomach. Most of ethanol is metabolized to acetaldehyde in liver by the following three pathways: alcohol dehydrogenase (ADH) pathway, microsomal ethanol oxidizing system (MEOS), and the catalase system. Acetaldehyde is primarily metabolized to acetate in liver by cytosolic ALDH1A1 and mitochondrial ALDH2 and ALDH1B1 (expressed in human but very slightly in mice). The body relies on these pathways and enzymes to eliminate ethanol and acetaldehyde from the blood after an individual has consumed alcohol. Otherwise, acetaldehyde, a highly reactive toxicant, may result in the tissue damage, the generation of reactive oxygen species and the formation of protein- and DNA-adducts.

Herein, it is demonstrated that intake of SF very slightly affected ethanol levels in the blood. On the other hand, elimination of acetaldehyde from the blood was accelerated in SF-fed mice, with an elimination constant roughly 2 times higher than that of control mice. In the SF-fed mice, enzyme activities of ALDH in cytosol/microsome and mitochondrial fractions, as well as the corresponding gene expressions of *Aldh1a1* and *Aldh2,* were significantly increased in the liver. In addition to these effects, intake of SF increased glutathione levels in liver (Toyama 2011), which can additionally contribute to the rapid elimination of acetaldehyde. Furthermore, increases in the enzyme activity of ALDH were observed in digestive organs including the stomach (fore- and glandular-stomach in mice) and upper intestine, which also demonstrated up-regulation of *Aldh1a1* and *Aldh2.*

ALDH3A1, a cytosolic ALDH mainly oxidizes medium chain aliphatic and aromatic aldehydes, and, to lesser extent, propionaldehyde. However, acetaldehyde is not a known substrate of ALDH3A1 (Santisteban 1985). The enzyme is found in cornea, stomach, and lung, but not in the liver. Herein it was demonstrated that basal expression of *Aldh3a1* was much higher in the stomach (fore- and glandular-stomach) than liver and upper intestine in CD-1 mice. When measured using propionaldehyde as a substrate, cytosolic/microsomal ALDH activity was increased in both parts of the stomach in SF-fed mice. This is partially due to other cytosolic ALDHs including ALDH1A1, but ALDH3A1 also contributed to the increase in the ALDH activity in the stomach.

ALDH3A1 is often referred as an inducible ALDH enzyme because it is readily induced by 2,3,7,8-tetrachlorodibenzo-p-dioxin (TCDD) (Vasillou 2004). In addition to the Aryl hydrocarbon receptor pathway that is mediated by TCDD, it has been shown that gene expression of *Aldh3a1* is regulated by the Keap1/Nrf2/ARE pathway (Alnouti 2008). 3) The presence of antioxidant response elements (AREs) in the 5 ' upstream region of ALDH has also been shown (Sreerama 2001). This resulted in induction of ALDH3A1 by intake of SF, but does not affect the acetaldehyde metabolism. However, up-regulation of ALDH3A1 contributes to the detoxification of toxic aldehydes.

Though not wishing to be bound by any particular theory, it is currently believed that induction of ALDHs, including ALDH1A1 and ALDH2 as well as a network of other phase 2 cytoprotective enzymes, is regulated by the Keap1/Nrf2/ARE pathway. Herein, various compounds were shown to be useful for their potencies for the induction of phase 2 enzymes, the acetaldehyde superfamily. Intake of plants including vegetables, fruits and herbs protect against acetaldehyde toxicity when drinking alcohol. Moreover, administration of phase 2 inducers, including sulforaphane, increased expression and activity of ALDHs, thereby reducing toxicities associated with the accumulation of acetaldehyde.

### Example 1 - Cell Culture

All cell lines were grown in 5% CO₂ at 37 °C. Murine hepatoma cell line, Hepa1c1c7 was cultured in α-MEM supplemented with 10% (v/v) heat inactivated FBS. Mouse embryo fibroblasts (MEFs) derived from day 13.5 embryos of wild-type or Nrf2-knockout C57BL/6 mice were cultured in Iscoves Modified Dulbecco's Medium (with L-glutamine) supplemented with human recombinant epidermal growth factor (10 ng/mL), 1 x insulin/transferring/selenium, and 10% (v/v) heat- inactivated FBS.

### Example 2 - Animal Experiments

All animal experiments were performed in compliance with National Institute of Health Guidelines and those of Animal Care and Use Committee of the Johns Hopkins University. In the first experiment, 15 female, 8-9 week-old CD-1 mice were used to assess the effects of dose of sulforaphane (SF) on enzyme activities and mRNA expressions of aldehyde dehydrogenases (ALDHs) in tissues. All mice were fed a low inducer-containing diet (AIN-76A) for 1 week, and were randomly assigned to 3 groups, control, low-, and high-SF group (n = 5 in each group). They received basal diet, or 5 or 20 µmol SF/3 g diet, respectively. After 1 week on these diets, mice were euthanized and their liver, forestomach, glandular stomach, and upper small intestine were harvested, frozen in liquid nitrogen, and stored at -80 °C until analyzed. A small portion of each tissue was stored in RNA stabilizing solution at 4 °C until total RNA extraction.

In the second experiment, whether SF affected blood ethanol and acetaldehyde levels was determined after a single dose of ethanol in female CD-1 mice. After acclimatization on the low inducer diet, mice were divided into two groups, control (n=9) and SF (n = 9), and then they received AΓN76A or 20 µmol SF per 3 g diet for 1 week, respectively. After fasting overnight, they were gavaged with 35% (v/v) ethanol solution at a dose of 2.0 g/kg. Before, and 0.5, 1, 2, 3, 4, and 6 h after administration of ethanol, 20 µL, of blood were collected by nicking the tail vein. The mice were then euthanized and their blood, liver, stomach and upper intestine were collected.

### Example 3 - Assay of Aldehyde Dehydrogenase (ALDH) Activity

To determine total ALDH activity, Hepa1c1c7 or MEF cells were plated into 6-cm plates at 6.0 x 10⁵ or 1.2 10⁶ cells/plate, incubated for 24 h at 37 °C in a 5% CO₂ atmosphere, and then treated with serial dilutions of inducers. The inducers were dissolved in acetonitrile or DMSO and added to medium so that final concentrations of the solvents was less than 0.5 or 0.1% v/v, respectively. After 48 h of treatment, cells were washed twice with PBS, scraped from the plates, and homogenized in micro-homogenizers at 4 °C. The homogenates were centrifuged at 5,000 g for 15 min at 4 °C and the supernatant fractions were collected. ALDH activity was measured fluorimetrically in 96-well plates (white-walled) by modification of the method of Koivula et al. (1975). The assay mixture (190 µL) contained 70 mM sodium pyrophosphate buffer (pH 8.0), 1.67 mM pyrazole, 1.33 mM NAD, and supernatant fraction. The assay was started by addition of 10 µL of 90 mM propionaldehyde, bringing the total volume to 200 µL. The initial velocity of NADH generation was measured (λₑₓ 340 nm and λₑₘ 460 nm) at 25 °C for 10 min in a micro-plate fluorometer. Blank reaction rates without propionaldehyde were also determined. The ALDH velocity was normalized to the protein concentration and expressed as a change in fluorescent units per min per mg of protein.

To determine ALDH activities of cytosolic/microsomal and mitochondrial fractions individually, cells or harvested tissues were homogenized at 4 °C in 10 mM Tris-HCI (pH 7.4) containing 0.25 M sucrose and 1 mM 2-mercaptoethanol and centrifuged at 750 x g for 15 min to remove nuclei and cell debris. Supernatant fractions were then centrifuged twice at 12,000 x g for 20 min to obtain the cytosolic/microsomal and the precipitated mitochondrial fractions. Mitochondrial fractions were suspended in 0.25 M sucrose, diluted with one-half volume of 1% sodium bicarbonate containing 1% sodium deoxycholate (w/v), and sonicated in a Branson Sonicator at 4 °C for five (5) 2-minute periods for a total of 10 min before assays.

### Example 4 - Assay of NAD(P)H-Quinone Acceptor Oxidoreductase (NQO1) Activity

The lysates of Hepa1c1c7 cells which were plated in 96-well plates at 10⁴ cells per well, grown for 24 h, and then exposed to serial dilutions of inducers for 48 h. Cytosolic/microsomal fractions of tissues were subjected to NQO1 assay. The enzyme activity of NQO1 was determined with menadione as substrate by the Prochaska assay (Prochaska 1988; Fahey 2004).

### Example 5 - Analysis of mRNA expression of ALDHs

Total RNA was extracted from Hepa1c1c7 cells that had been exposed to serial dilutions of inducers for 24 h, or from RNA-stabilized tissues (stored in TRizol) from mice, followed by reverse transcription into cDNA. The cDNA was subjected to quantitative realtime PCR analysis by using SYBR Green fluorescence with 7000 Sequence Detection System. All primers used in this study were designed based on previous reports. Relative expressions of individual mRNAs for ALDH were normalized to β-actin as an endogenous control, and the expression rate was calculated using the comparative ΔΔCt method.

### Example 6 - Determinations of Ethanol and Acetaldehyde Levels in Mouse Blood

Blood ethanol and acetaldehyde levels in mice were determined enzymatically by modification of the method of Tottmar et al. (Tottmar 1978). Whole blood samples (20 µL) were obtained by repeated incisions of mouse tail veins. The blood was immediately mixed with 200 µL of 4% (v/v) perchloric acid (PCA) in sealed tubes at 4 °C, incubated for 10 min, and then centrifuged at 3000 x g for 10 min. The pH values of the supernatant fractions were adjusted to 7.5 - 8.0 by adding 20 µL of 3 M K₂CO₃ in new tubes. After removing precipitated KClO₄ by centrifugation at 3000 x g for 10 min, supernatant fractions were stored in sealed tubes at 4 °C until assayed.

Diluted supernatant fractions were added to each well of 96-well plates containing in a final volume of 200 µL: 70 mM sodium pyrophosphate buffer (pH 9.0), 0.6 mM NAD, and 53 units/mL of alcohol dehydrogenase (purified ADH from yeast). Blood ethanol level was calculated from the rate of NADH generation for 5 min measured with a micro-plate fluorometer (λₑₓ 340 nm and λₑₘ 460 nm) by using ethanol calibration curve.

Determination of acetaldehyde was performed in 10 mm quarts fluorescence cuvettes in a final volume of 2.33 mL assay mixture containing of assay mixture containing 50 mM sodium pyrophosphate (pH 8.8), 0.1 mM pyrazole, 0.1 mM NAD and 0.2 units/mL of purified ALDH (potassium activated from baker's yeast) with a luminescence spectrophotometer. Before and 5 min after addition of 170 µL of the supernatant, intensities were monitored (λₑₓ: 340 nm and λem: 460 nm). The acetaldehyde level in blood was calculated from change of intensity using acetaldehyde standard curve with range from 1.56 to 50 µM in blood. The standard curve was made by applying serial dilution of acetaldehyde to PCA treatment process same as blood.

### Example 7 - Effect of Sulforaphane on Total ALDH Activity in Hepa1c1c7 Cells

The effect of sulforaphane (SF) on total ALDH activity in Hepa1c1c7 cells was assessed using an established ALDH assay system comprising a micro-plate fluorometer. Cells were treated with a series of concentrations (0.1, 0.3, 1.0 and 3.0 µM) of SF for 48 hours. Treatment dramatically and dose dependently increased total ALDH activity, which includes cytosolic-, microsomal- and mitochondrial-ALDH activity in Hepa1c1c7 cells. Significant effects were observed in cells treated with 0.3 µM or higher concentrations of SF (FIG. 1A). The total ALDH activity in Hepa1c1c7 cells was not increased in first 12 h when treated with 3 µM SF, but remarkably increased up to 1.5, 1.9 and 2.1 fold above control levels following an additional 6, 12 and 24 hours, respectively (FIG. 1B). This result shows that SF may induce ALDH but not directly activate it.

### Example 8 - Effects of Various Compounds with Different Chemical Structures on ALDH Activity in Hepa1c1c7 Cells

To determine structure-activity relationships, 21 compounds (15 phyto-chemicals and 6 synthetic compounds) belonging to 8 structurally distinct chemical classes were chosen for further screening. Most of the compounds tested have been reported previously as phase 2 inducers (Table 1). As a result of assessment of these compounds, 16 compounds, including SF, increased total ALDH activity in Hepa1c1c7 cells in a dose dependent manner. Observed increases in activity were noted in compounds belonging to each of the 8 chemical classes (FIG. 2), indicating the effects were elicited independent of compound structure.

### Example 9 - Relationship of Potencies of Compounds for Induction of ALDH and NQO1 in Hepa1c1c7 Cells

To investigate the involvement of phase 2 inducers on ALDH activity, potencies of these compounds for the induction of NQO1 and ALDH were estimated as CD values, which represent concentrations required to double the enzyme activity of an enzyme of interest (Table 1). The CD values were distributed in more than 5 orders magnitude of concentrations over the 8 chemical classes, ranging from the most potent, triterpenoid TP-225, with a CD value of 0.0016 µM for ALDH and 0.00038 µM for NQO1, to the least potent compound assessed, stilbenoids resveratrol, with CD values of 120.7 µM and >50 µM, respectively. All CD values for induction of ALDH were uniformly 5-10 times higher than those for induction of NQO1, which implies ALDHs are less sensitive to phase 2 inducers compared to NQO1 in Hepa1c1c7 cells.

To further illustrate the relationship of CD values for induction of ALDH and NQO1, CD values of 16 compounds were plotted on a double logarithmic chart (FIG. 3). A linear analysis highlights the excellent correlation of potencies of the 16 compounds for induction of ALDH and NQO1 in Hepa1c1c7 cells, where the correlation was observed over more than 5 orders of magnitude with an r² value of 0.94 and a slope of 0.87. This clearly shows that induction of ALDH by phase 2 inducers is elicited through the same pathway for induction of NQO1, namely Keap1/Nrf2/ARE pathway.

### Example 10 - Participation of Nrf2 in Induction of ALDH by SF

To determine whether Nrf2 participates in induction of ALDH or not, effects of a stepwise concentration of SF on total ALDH activity were tested in WT and Nrf2^{-/-} MEFs. Although the sensitivity of total ALDH activity to SF was less than that of Hepa1c1c7 cells, the activity increased in a dose dependent manner for treatment of WT MEFs with SF. On the other hand, the effect of SF was altogether absent in Nrf2⁻¹⁻ MEFs (FIG. 4). This result demonstrates that Nrf2 is involved in the induction of ALDH by SF and possibly other phase 2 inducers.

### Example 11 - Effects of Phase 2 on Induction of Individual ALDH isothiocyanate

Three representative phase 2 inducers with different structures, isothiocyanate SF (1 or 3 µM), triterpenoid TP-225 (0.003 or 0.01 µM) and flavonoid BNF (0.3 or 1 µM) were assessed for their effect on induction of individual ALDH, particularly cytosolic ALDH1A1 and ALDH3A1 and mitochondrial ALDH2, which are primarily responsible for acetaldehyde metabolism. Cytosolic ALDH3A1 are known as an inducible ALDH because they can be induced by xenobiotics including dioxin. ALDH3A1 are expressed in stomach cells and hepatoma such as Hepa1c1c7 cells, despite of having little or no expression in normal hepatic cells. The cells were treated with the inducers for 24 hours, resulting in a dose dependent elevation of mRNA expressions of individual ALDH genes, *Aldh1a1, Aldh2* and Aldh3*a1* in Hepa1c1c7 cells (FIG. 5). As for *Aldh1a1* and *Aldh2,* the mRNA expression levels increased significantly, reaching up to 1.2-1.5 fold higher than control. The *Aldh3a1* was most sensitive, showing around a 5-fold increase from each inducer, although the basal expression level, normalized to endogenous β-actin, was more than 20 and 10 times lower than that of *Aldh1a1* and *Aldh2,* respectively. These results indicated that phase 2 inducers induced individual ALDH genes including at least *Aldh1a1, Aldh2* and *Aldh3a1*; thus the Nrf2 pathway is plays a role in the expressions of individual ALDH genes.

### Example 12 - Antioxidant Response Elements (ARE) Consensus Sequences in ALDH Genes

Sulforaphane induces the expression of ALDH genes such as *Aldh1a1, Aldh2* and *Aldh3a1.* Among these genes, *Aldh1a1* and *Aldh2* are known to code for enzymes that are responsible for acetaldehyde metabolism, with *Aldh3a1* mainly metabolizing aromatic aldehydes. Previous reports suggest that induction of *Aldh1a1* and *Aldh3a1* is mediated by the Keap1/Nrf2/ARE pathway because ARE core consensus sequences were identified in the 5'-flanking region of these genes (Sreema 2001; Abdullah 2012). However it has never been determined whether *Aldh2* contains the ARE sequence or not. Thus, identification of the ARE core consensus sequence, RTGA(S/Y)nnnGCR (where R = A or G, S = C or G, Y = C or T and n = any nucleotide), was performed by established methods (Abdullah 2012). At least two perfect matches (8/8) of the sequences were found within 2,000-bp of 5'-flanking region of *Aldh2* that was obtained from the *ENSEMBL* mouse project website. The positions of perfect sequences, GTGACcagGCG and ATGAGacaGCA are situated 82-92 bp and 1364-1374 bp upstream of the putative transcription start site, respectively. Both ARE sequences are categorized as class 4 enhancers because they are not embedded in an activator protein 1-binding site TGASTCA (Hayes 2010). Additionally, more than 10 similar sequences (scored 5/8-7/8) were found in the region. This finding indicates that the induction of *Aldh2* is likely to be mediated by the Keap1/Nrf2/ARE pathway.

### Example 13 Effects of Sulforaphane on ALDH Activity in Mice

To evaluate potential of sulforaphane for induction of ALDH *in vivo,* CD-1 mice received 2 doses of SF, either 5 (low-dose) or 20 µmol (high-dose), per 3 grams of diet per mouse per day for 1 week. Importantly, there were no significant differences in body weights and food intakes among these groups throughout the feeding period. Following the 1 week treatment, ALDH activity was measured in cytosolic/microsomal and mitochondrial cell fractions, and mRNA expression of ALDHs in tissues (liver, fore- and glandular-stomachs, and upper intestine) was determined. Intake of SF for 1 week resulted in a dose dependent increase in NQO1 activity in the liver, both parts of the stomach, and upper intestine, reaching up to a 2.2-fold increase in activity compared to control in the high dose group (FIG. 6).

As for ALDH, both cytosolic/microsomal and mitochondrial-ALDH activities were significantly increased by 1.4- and 1.5-fold in the liver as a result of the high dose of SF. This is of note because the liver plays a more important role than any other organ in metabolizing acetaldehyde derived from ethanol. The basal activity of mitochondrial ALDH in liver was higher than other tissues. Also intake of SF dose dependently increased ALDH activities in both fractions in other tissues. Strikingly, the highest sensitivity to SF was observed in glandular-stomach where cytosolic/microsomal and mitochondrial ALDH activities were increased up to 4.1-(low) and 5.1- fold (high) above control. The basal mitochondrial activity in stomach was 5-10 times lower than that in liver, whereas there was no significant difference in the basal cytosolic/microsomal ALDH activities in stomach and liver.

Next, mRNA expression of ALDH genes was analyzed in mice. SF increased the expression levels of ALDH genes including *Aldh1a1, Aldh2* and *Aldh3a1* in all tissues examined (FIG. 7), but the basal expression levels of these genes varied between tissues. The expression of Aldh*1a1* and *Aldh2* were extremely high in the liver compared to other tissues, whereas that of *Aldh3a1* was expressed almost exclusively in both parts of the stomach, and only very slightly in the liver and the upper intestine. In the liver, expression levels of *Aldh1a1* and *Aldh2* were increased by 2.5- and 1.8-fold, respectively, which likely contributes to the increase in ALDH activity observed in the cytosolic/microsomal- and mitochondrial fractions of mouse liver. In the stomach, *Aldh3a1* expression increased by 3-fold in the forestomach and 6.5-fold in the glandular stomach. Since the highest levels of induction of *Aldh3a1* were observed in the glandular stomach, ALDH3A1 is likely responsible of much of the acetaldehyde dehydrogenase activity in glandular stomach. In upper intestine, basal expression levels were generally lower than in other tissues and little induction was observed. These results show that sulforaphane induces of ALDHs as well as phase 2 enzymes, including NQO1, *in vivo.* Thus, sulforaphane prevents acetaldehyde toxicities by enhancing acetaldehyde metabolism and inducing ALDH.

### Example 14 - Effect of Intake of SF on Blood Ethanol and Acetaldehyde Levels in Mice

To demonstrate effects of SF on alcohol metabolisms *vivo,* changes in blood ethanol and acetaldehyde levels were measured in CD-1 mice that received 20 µmol of SF per 3 g diet per day per mouse for 1 week prior to a single oral administration of 2.0 g/kg ethanol.

In control mice, the blood ethanol and acetaldehyde levels increased up to approximately 30 mM and 22 µM at 1 to 2 h after ethanol gavage, respectively. After this time, ethanol was mostly undetectable but acetaldehyde was still remaining in blood at 6 hours after the ethanol gavage. Intake of SF slightly affected the ethanol level, but only in the descending phase of blood ethanol curve. However, there was no significant difference in between the area under the curves (AUCs) for ethanol detection in control and SF fed mice. In contrast, intake of SF remarkably prevented increase in blood acetaldehyde level by 30% in the ascending phase, and kept it at a lower level throughout the detection period. Compared to control mice (83.8 ± 6.1 µmol•h/L), SF significantly reduced the AUC of blood acetaldehyde to 54.5 ± 5.1 µmol•h/L. Pharmacokinetic analysis using a first order model revealed that elimination of blood acetaldehyde was prominently accelerated in SF fed mice compared to control mice with 1.77 ± 0.12 and 3.43 ± 0.23 h of half-lives, respectively (Elimination rate constants, k: 0.40 ± 0.03 h⁻¹ and 0.21 ± 0.02 h⁻¹, p<0.01, Initial blood levels, C₀: 32.38 ± 2.4 µM and 28.62 ± 2.1 µM, NS). Thus, sulforaphane prevents acetaldehyde toxicities by enhancing acetaldehyde metabolism and inducing ALDH.

## Claims

1. A composition comprising a compound which modulates acetaldehyde dehydrogenase such that the activity of said acetaldehyde dehydrogenase is increased for use in preventing, treating or reducing alcohol toxicity, wherein said compound is one or more of sulforaphane, erucin, iberin, benzyl ITC, phenethyl ITC, propyl ITC, hexyl ITC, 4RBITC, TP-225, celastrol, withaferin A, TBE-31, HBB-2, HBB-4, BNF, pinostrobin, tectochrisin, kaempferide, resveratrol and/or zerumbone.

2. A composition for use according to claim 1, wherein said compound induces ALDH and NQO1.

3. A composition for use according to claim 1 or 2, wherein said compound is sulforaphane.

4. A composition for use according to any one of claims 1-2, wherein the compound is present as a structure represented by a formula shown below, or a subgroup or pharmaceutically acceptable salts thereof, 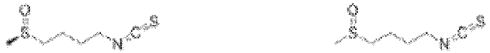 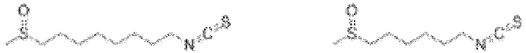 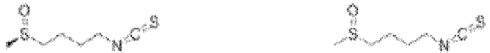 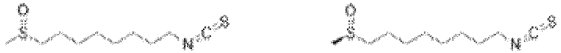 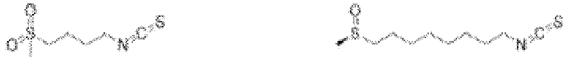 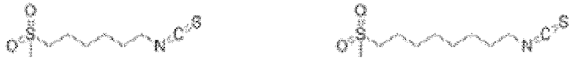 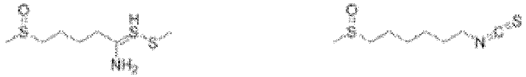 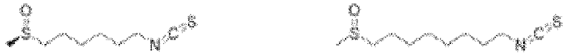 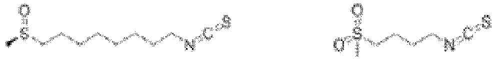 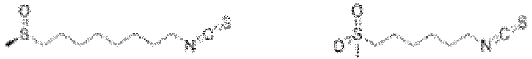 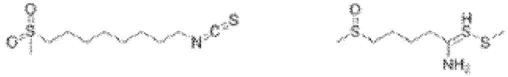 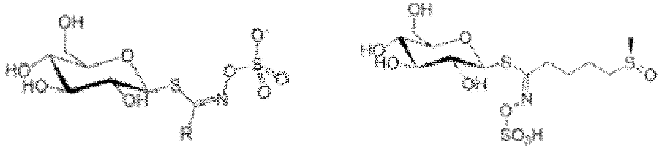 or

5. A composition for use according to any one of the preceding claims, wherein said compound which modulates acetaldehyde dehydrogenase is sulforaphane.

6. A composition for use according to any one of the preceding claims, wherein said preventing, treating or reducing alcohol toxicity reduces the risk of cancer due to acetaldehyde exposure, such as esophageal cancer.

7. A composition for use according to any one of the preceding claims, wherein the composition is a dietary supplement.

8. A composition for use according to any one of claims 1-6, wherein the composition is a nutraceutical.

9. A composition for use according to any one of claims 1-6, wherein the composition is a pharmaceutical composition.

10. A composition for use according to any one of claims 1-6, wherein the composition further comprises a pharmaceutically acceptable carrier.

11. A composition for use according to any one of claims 1-6, wherein the composition is a medicament.

12. A composition for use according to any one of claims 1-11, wherein the subject consumes ethanol.

13. A composition for use according to any one of claims 1-11, wherein the composition is administered to the subject before the subject consumes ethanol.

14. A composition for use according to any one of the preceding claims, wherein the composition is in a form selected from the group consisting of tablet form, liquid form, powder form, capsule form, injectable form and spray form.

15. A method for in vitro reducing acetaldehyde levels and/or increasing the rate of catabolism of acetaldehyde, comprising, contacting at least one cell with an effective amount of a compound that modulates the enzymatic activity of an acetaldehyde dehydrogenase found in at least one cell, wherein said compound is a compound as defined in any one of claims 1-6.

## Patentansprüche

1. Zusammensetzung, umfassend eine Verbindung, die Acetaldehyddehydrogenase moduliert, sodass für die Verwendung zur Prävention, Behandlung oder Reduzierung von Alkoholtoxizität die Aktivität der Acetaldehyddehydrogenase erhöht wird, wobei die Verbindung eines oder mehrere aus Sulforaphan, Erucin, Iberin, Benzyl-ITC, Phenethyl-ITC, Propyl-ITC, Hexyl-ITC, 4RB-ITC, TP 225, Celastrol, Withaferin A, TBE 31, HBB 2, HBB 4, BNF, Pinostrobin, Tectochrisin, Kaempferid, Resveratrol und/oder Zerumbon ist.

2. Zusammensetzung für die Verwendung nach Anspruch 1, wobei die Verbindung ALDH und NQO1 induziert.

3. Zusammensetzung für die Verwendung nach Anspruch 1 oder 2, wobei die Verbindung Sulforaphan ist.

4. Zusammensetzung für die Verwendung nach einem der Ansprüche 1-2, wobei die Verbindung als eine Struktur vorliegt, die durch eine unten gezeigte Formel dargestellt wird, oder eine Untergruppe oder pharmazeutisch verträgliche Salze davon,

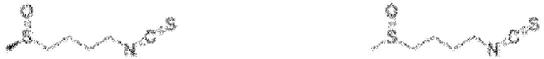

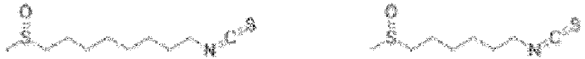

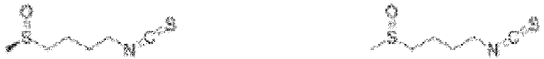

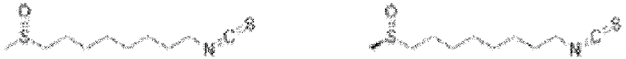

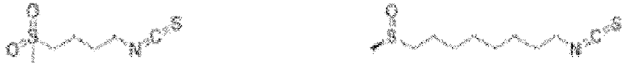

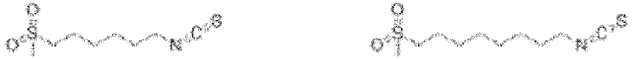

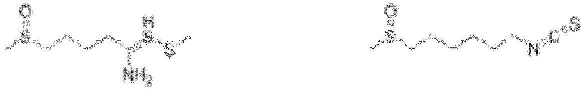

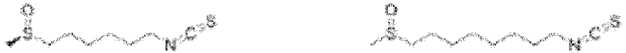

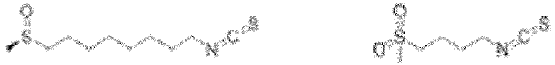

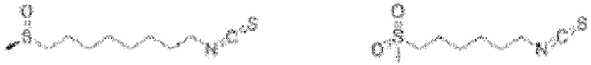

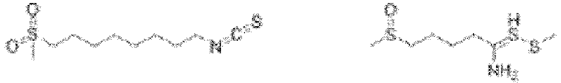

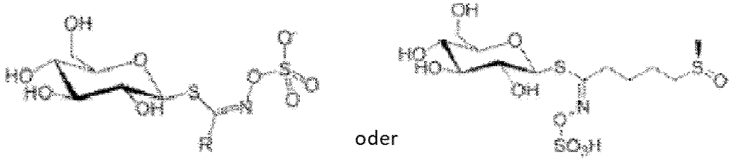

5. Zusammensetzung für die Verwendung nach einem der vorstehenden Ansprüche, wobei die Verbindung, die Acetaldehyddehydrogenase moduliert, Sulforaphan ist.

6. Zusammensetzung für die Verwendung nach einem der vorstehenden Ansprüche, wobei die Prävention, Behandlung oder Reduzierung von Alkoholtoxizität das Risiko von Krebs infolge von Acetaldehydexposition, wie Speiseröhrenkrebs, verringert.

7. Zusammensetzung für die Verwendung nach einem der vorstehenden Ansprüche, wobei die Zusammensetzung ein Nahrungsergänzungsmittel ist.

8. Zusammensetzung für die Verwendung nach einem der Ansprüche 1-6, wobei die Zusammensetzung ein Nutrazeutikum ist.

9. Zusammensetzung für die Verwendung nach einem der Ansprüche 1-6, wobei die Zusammensetzung ist eine pharmazeutische Zusammensetzung ist.

10. Zusammensetzung für die Verwendung nach einem der Ansprüche 1-6, wobei die Zusammensetzung weiter einen pharmazeutisch verträglichen Träger umfasst.

11. Zusammensetzung für die Verwendung nach einem der Ansprüche 1-6, wobei die Zusammensetzung ein Medikament ist.

12. Zusammensetzung für die Verwendung nach einem der Ansprüche 1-11, wobei das Subjekt Ethanol konsumiert.

13. Zusammensetzung für die Verwendung nach einem der Ansprüche 1-11, wobei die Zusammensetzung dem Subjekt verabreicht wird, bevor das Subjekt Ethanol konsumiert.

14. Zusammensetzung für die Verwendung nach einem der vorstehenden Ansprüche, wobei die Zusammensetzung in einer Form vorliegt, die ausgewählt ist aus der Gruppe bestehend aus Tablettenform, flüssiger Form, Pulverform, Kapselform, injizierbarer Form und Sprühform.

15. Verfahren zur In-vitro-Reduzierung von Acetaldehydgehalten und/oder - Steigerung der Rate des Acetaldehyd-Katabolismus, umfassend Inkontaktbringen mindestens einer Zelle mit einer wirksamen Menge einer Verbindung, die die enzymatische Aktivität einer Acetaldehyddehydrogenase moduliert, die in mindestens einer Zelle gefunden wird, wobei die Verbindung eine Verbindung ist, wie sie in einem der Ansprüche 1-6 definiert ist.

## Revendications

1. Composition comprenant un composé qui module de l'acétaldéhyde déshydrogénase de sorte que l'activité de ladite acétaldéhyde déshydrogénase soit augmentée pour utilisation dans la prévention, le traitement ou la réduction de la toxicité alcoolique, dans laquelle ledit composé est un ou plusieurs parmi le sulforaphane, l'érucine, l'ibérine, le benzyle ITC, le phénéthyle ITC, le propyle ITC, l'hexyle ITC, le 4RBITC, le TP-225, le célastrol, la withaferin A, le TBE-31, le HBB-2, le HBB-4, le BNF, le pinostrobin, le tectochrisin, le kaempféride, le resvératrol et/ou le zerumbone.

2. Composition pour utilisation selon la revendication 1, dans laquelle ledit composé induit de l'ALDH et du NQO1.

3. Composition pour utilisation selon la revendication 1 ou 2, dans laquelle ledit composé est du sulforaphane.

4. Composition pour utilisation selon l'une quelconque des revendications 1 à 2, dans laquelle le composé est présent comme une structure représentée par une formule indiquée ci-dessous, ou un sous-groupe ou des sels pharmaceutiquement acceptables de ceux-ci, 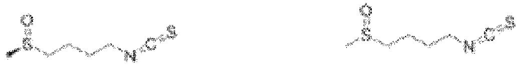 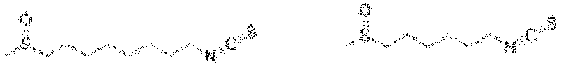 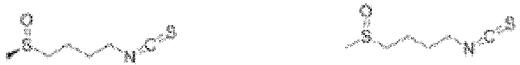 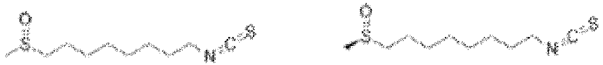 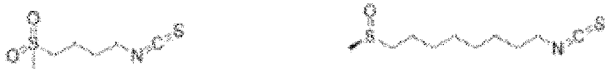 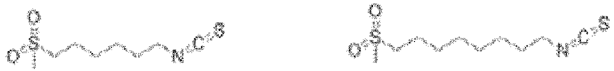 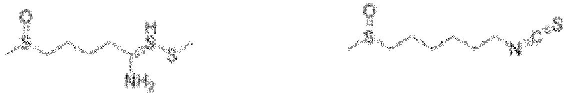 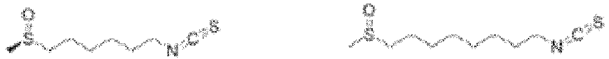 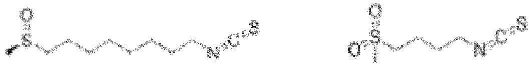 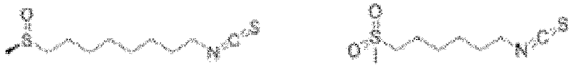 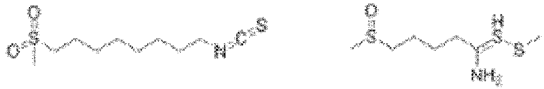 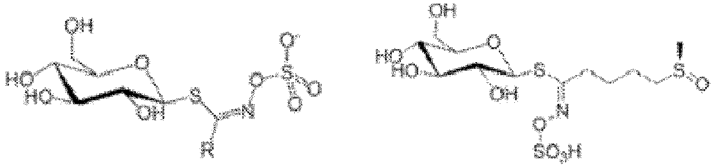 ou

5. Composition pour utilisation selon l'une quelconque des revendications précédentes, dans laquelle ledit composé qui module de l'acétaldéhyde déshydrogénase est du sulforaphane.

6. Composition pour utilisation selon l'une quelconque des revendications précédentes, dans laquelle ladite/ledit prévention, traitement ou réduction de la toxicité alcoolique réduit le risque de cancer en raison d'une exposition à l'acétaldéhyde, comme le cancer oesophagien.

7. Composition pour utilisation selon l'une quelconque des revendications précédentes, dans laquelle la composition est un complément alimentaire.

8. Composition pour utilisation selon l'une quelconque des revendications 1 à 6, dans laquelle la composition est un produit nutraceutique.

9. Composition pour utilisation selon l'une quelconque des revendications 1 à 6, dans laquelle la composition est une composition pharmaceutique.

10. Composition pour utilisation selon l'une quelconque des revendications 1 à 6, dans laquelle la composition comprend en outre un support pharmaceutiquement acceptable.

11. Composition pour utilisation selon l'une quelconque des revendications 1 à 6, dans laquelle la composition est un médicament.

12. Composition pour utilisation selon l'une quelconque des revendications 1 à 11, dans laquelle le sujet consomme de l'éthanol.

13. Composition pour utilisation selon l'une quelconque des revendications 1 à 11, dans laquelle la composition est administrée au sujet avant que le sujet ne consomme de l'éthanol.

14. Composition pour utilisation selon l'une quelconque des revendications précédentes, dans laquelle la composition est sous une forme sélectionnée à partir du groupe constitué par une forme de comprimé, une forme liquide, une forme de poudre, une forme de capsule, une forme injectable et une forme de vaporisateur.

15. Procédé pour réduire in vitro des niveaux d'acétaldéhyde et/ou augmenter la vitesse de catabolisme d'acétaldéhyde, comprenant la mise en contact d'au moins une cellule avec une quantité efficace d'un composé qui module l'activité enzymatique d'un acétaldéhyde déshydrogénase trouvé dans au moins une cellule, dans lequel ledit composé est un composé tel que défini selon l'une quelconque des revendications 1 à 6.
